(19) Europäisches Patentamt
European Patent Office
Office européen des brevets

(11) EP 1 352 659 A1

(12) EUROPEAN PATENT APPLICATION
published in accordance with Art. 158(3) EPC

(43) Date of publication:
15.10.2003 Bulletin 2003/42

(21) Application number: 01271853.2

(22) Date of filing: 21.12.2001

(51) Int Cl.[7]: A61K 45/06, A61K 31/551, A61K 31/221, A61K 31/4468, A61P 11/00, A61P 29/00, A61P 19/02, A61P 11/14, A61P 1/00, A61P 1/08, A61P 25/24, A61P 25/22, A61P 25/18, A61P 43/00

(86) International application number:
PCT/JP01/11231

(87) International publication number:
WO 02/051440 (04.07.2002 Gazette 2002/27)

(84) Designated Contracting States:
AT BE CH CY DE DK ES FI FR GB GR IE IT LI LU MC NL PT SE TR
Designated Extension States:
AL LT LV MK RO SI

(30) Priority: 22.12.2000 JP 2000391013

(71) Applicant: Takeda Chemical Industries, Ltd.
Osaka-shi, Osaka 541-0045 (JP)

(72) Inventors:
- DOI, Takayuki
Osaka-shi, Osaka 545-0042 (JP)
- HASHIMOTO, Tadatoshi
Ibaraki-shi, Osaka 567-0828 (JP)
- KAMO, Izumi
Amagasaki-shi, Hyogo 661-0047 (JP)

(74) Representative:
von Kreisler, Alek, Dipl.-Chem. et al
Patentanwälte
von Kreisler-Selting-Werner
Postfach 10 22 41
50462 Köln (DE)

(54) **COMBINATION DRUGS**

(57) A pharmaceutical agent containing an NK-1 receptor antagonist, an NK-2 receptor antagonist and/or an anti-cholinergic drug in combination is provided, which is useful as a prophylactic or therapeutic agent of urinary frequency, urinary incontinence, asthma, chronic obstructive pulmonary disease, rheumatoid arthritis, osteoarthritis, pain, cough, irritable bowel syndrome, emesis, depression, anxiety, manic depressive psychosis or schizophrenia. More particularly, a pharmaceutical agent is provided, which contains a compound represented by the formula (I), wherein M ring is a heterocyclic ring having -N=C<, -CO-N< or -CS-N< as a partial structure — X ------ Y < ; $R^a$ and $R^b$ are bonded to each other to form ring A, or the same or different and each is a hydrogen atom or a substituent for ring M; ring A and ring B are each a homocyclic or heterocyclic ring optionally having substituents and at least one of them is a heterocyclic ring optionally having substituents; ring C is a homocyclic or heterocyclic ring optionally having substituents; ring Z is an optionally substituted heterocyclic ring containing nitrogen; and n is an integer of 1 to 6, or a salt thereof or a prodrug thereof, and an NK-2 receptor antagonist and/or an anti-cholinergic drug in combination.

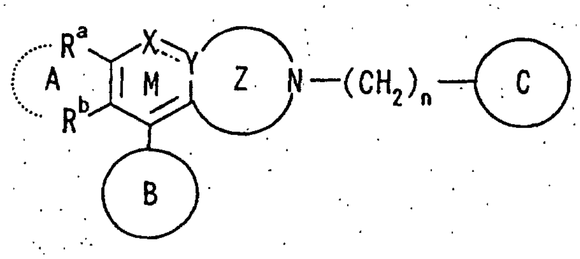

(I)

EP 1 352 659 A1

## Description

### Technical Field

The present invention relates to a pharmaceutical agent comprising an NK-1 receptor antagonist, an NK-2 receptor antagonist and/or an anti-cholinergic drug in combination.

### Background Art

JP-A-9-263585 describes that compound (I) represented by the formula

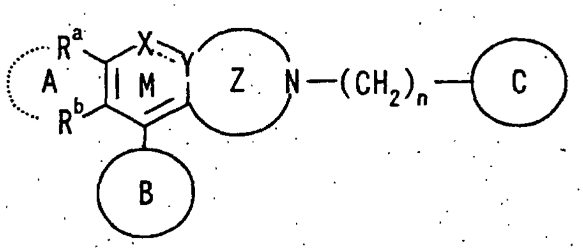

(I)

wherein ring M is a heterocyclic ring having -N=C<, -CO-N< or - CS-N< as a partial structure — X ------ Y <;
$R^a$ and $R^b$ are bonded to each other to form ring A, or the same or different and each is a hydrogen atom or a. substituent on ring M;
ring A and ring B are each a homocyclic or heterocyclic ring optionally having substituents, wherein at least one of them is a heterocyclic ring optionally having substituents;
ring C is a homocyclic or heterocyclic ring optionally having substituents;
ring Z is an optionally substituted heterocyclic ring containing nitrogen; and
n is an integer of 1 to 6, or a salt thereof has a tachykinin receptor antagonistic action, a substance P receptor antagonistic action, and a neurokinin A receptor antagonistic action.

### Disclosure of the Invention

The present invention aims at providing a pharmaceutical agent that can be widely applied to the diseases such as urinary frequency, urinary incontinence, asthma, chronic obstructive pulmonary disease, rheumatoid arthritis, osteoarthritis, pain, cough, irritable bowel syndrome, emesis, depression, anxiety, manic depressive psychosis, schizophrenia and the like.

In view of the above-mentioned situation, the present inventors have conducted intensive studies and found that a combined use of an NK-1 receptor antagonist, particularly, the above-mentioned compound (I) described in JP-A-9-263585 or a salt thereof or a prodrug thereof, an NK-2 receptor antagonist and/or an anti-cholinergic drug leads to

(1) provision of a superior treatment effect on diseases such as urinary frequency, urinary incontinence, asthma, chronic obstructive pulmonary disease, rheumatoid arthritis, osteoarthritis, pain, cough, irritable bowel syndrome, emesis, depression, anxiety, manic depressive psychosis, schizophrenia and the like and
(2) decreased dose of an anti-cholinergic drug or an NK-2 receptor antagonist as compared to a single administration thereof. The present inventors have conducted further studies based on these findings, which resulted in the completion of the present invention.

Accordingly, the present invention provides

[1] a pharmaceutical agent comprising a compound (I) represented by the formula

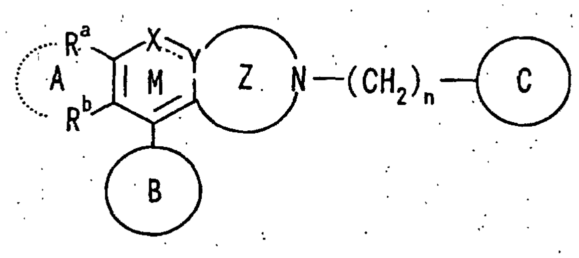

wherein

| | |
|---|---|
| ring M | is a heterocyclic ring having -N=C<, -CO-N< or -CS-N< as a partial structure - X ------ Y < ; |
| $R^a$ and $R^b$ | are bonded to each other to form ring A, or the same or different and each is a hydrogen atom or a substituent on ring M; |
| ring A and ring B | are each a homocyclic or heterocyclic ring optionally having substituents, wherein at least one of them is a heterocyclic ring optionally having substituents; |
| ring C | is a homocyclic or heterocyclic ring optionally having substituents; |
| ring Z | is an optionally substituted heterocyclic ring containing nitrogen; and |
| n | is an integer of 1 to 6, |

or a salt thereof or a prodrug thereof and an NK-2 receptor antagonist in combination,
[2] the pharmaceutical agent of the above-mentioned [1], wherein the compound (I) is

(i) (9S)-7-[3,5-bis(trifluoromethyl)benzyl]-6,7,8,9,10,12-hexahydro-9-methyl-6,12-dioxo-5-phenyl[1,4]diazepino[2,1-g] [1,7]naphthyridine,
(ii) (9S)-7-[3,5-bis(trifluoromethyl)benzyl]-6,7,8,9,10,12-hexahydro-9-methyl-5-(4-methylphenyl)-6,12-dioxo [1,4]diazepino[2,1-g] [1,7]naphthyridine,
(iii) (9R)-7-[3,5-bis(trifluoromethyl)benzyl]-6,7,8, 9,10,11-hexahydro-9-methyl-6,13-dioxo-5-phenyl-13H-[1,4] diazocino[2,1-9][1,7]naphthyridine,
(iv) (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine,
(v) (9R)-7-(3,5-dimethoxybenzyl)-5-(4-fluorophenyl)-6,7,8,9,10,11-hexahydro-9-methyl-6,13-dioxo-13H-[1,4] diazocino[2,1-g][1,7]naphthyridine or
(vi) (9R)-7-(3,5-dimethoxybenzyl)-6,7,8,9,10,11-hexahydro-9-methyl-5-(4-methyiphenyl)-6,13-dioxo-13H-[1,4]diazocino(2,1-g][1,7]naphthyridine,

[3] the pharmaceutical agent of the above-mentioned [1], wherein the compound (I) is (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7] naphthyridine,
[4] the pharmaceutical agent of the above-mentioned [1], wherein the NK-2 receptor antagonist is a piperidine derivative, a perhydroisoindole derivative, a quinoline derivative, a pyrrolopyrimidine derivative or a pseudopeptide derivative,
[5] the pharmaceutical agent of the above-mentioned [1], which is GR94800, GR159897, MEN10627, MEN11420 (nepadutant), SR144190, SR48968 (saredutant), GR149861, YM35375/YM38336, ZD7944, L-743986, MDL105212A, ZD6021, MDL105172A, SCH205528, SCH62373, R-113281, RPR-106145, SB-414240, ZM-253270, SCH217048, L-659877, PD-147714 (CAM-2291), MEN10376, 516474, GR100679, DNK333, GR94800, UK-224671, MEN10376 or a salt thereof,
[6] the pharmaceutical agent of the above-mentioned [1], which is a prophylactic or therapeutic agent of urinary frequency, urinary incontinence, asthma, chronic obstructive pulmonary disease, rheumatoid arthritis, osteoarthritis, pain, cough, irritable bowel syndrome, emesis, depression, anxiety, manic depressive psychosis or schizophrenia,
[7] a pharmaceutical agent comprising an NK-1 receptor antagonist, and NK-2 receptor antagonist and an anticholinergic drug in combination,
[8] a prophylactic or therapeutic agent of urinary frequency or urinary incontinence, which comprises an NK-1 receptor antagonist and an NK-2 receptor antagonist in combination,
[9] a method for the prophylaxis or treatment of urinary frequency, urinary incontinence, asthma, chronic obstructive pulmonary disease, rheumatoid arthritis, osteoarthritis, pain, cough, irritable bowel syndrome, emesis, depression,

anxiety, manic depressive psychosis or schizophrenia, which comprises administering, to a mammal, an effective amount of compound (I) represented by the formula

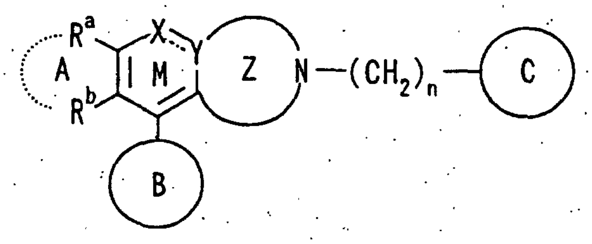

wherein

| | |
|---|---|
| ring M | is a heterocyclic ring having -N=C<, -CO-N< or -CS-N< as a partial structure —X ------ Y <; |
| $R^a$ and $R^b$ | are bonded to each other to form ring A, or the same or different and each is a hydrogen atom or a substituent on ring M; |
| ring A and ring B | are each a homocyclic or heterocyclic ring optionally having substituents, wherein at least one of them is a heterocyclic ring optionally having substituents; |
| ring C | is a homocyclic or heterocyclic ring optionally having substituents; |
| ring Z | is an optionally substituted heterocyclic ring containing nitrogen; and |
| n | is an integer of 1 to 6, |

or a salt thereof or a prodrug thereof and an effective amount of an NK-2 receptor antagonist in combination,
[10] use of compound (I) represented by the formula

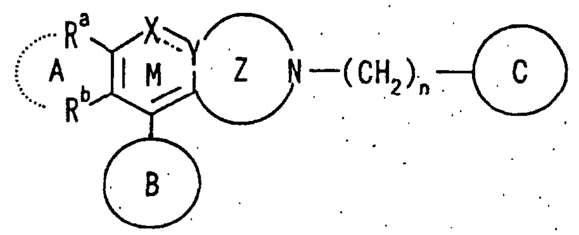

wherein

| | |
|---|---|
| ring M | is a heterocyclic ring having -N=C<, -CO-N< or -CS-N< as a partial structure -X ------ Y < ; |
| $R^a$ and $R^b$ | are bonded to each other to form ring A, or the same or different and each is a hydrogen atom or a substituent on ring M; |
| ring A and ring B | are each a homocyclic or heterocyclic ring optionally having substituents, wherein at least one of them is a heterocyclic ring optionally having substituents; |
| ring C | is a homocyclic or heterocyclic ring optionally having substituents; |
| ring Z | is an optionally substituted heterocyclic ring containing nitrogen; and |
| n | is an integer of 1 to 6, |

or a salt thereof or a prodrug thereof and an NK-2 receptor antagonist, for the production of a prophylactic or therapeutic agent of urinary frequency, urinary incontinence, asthma, chronic obstructive pulmonary disease, rheumatoid arthritis, osteoarthritis, pain, cough, irritable bowel syndrome, emesis, depression, anxiety, manic depressive psychosis or schizophrenia,
[11] a pharmaceutical agent comprising (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridin and an anti-cholinergic drug in combination,
[12] the pharmaceutical agent of the above-mentioned [7] or [11], wherein the anti-cholinergic drug is oxybutynin, propiverine, darifenacin, tolterodine, temiverine, trospium chloride or a salt thereof,
[13] the pharmaceutical agent of the above-mentioned [11], which is a prophylactic or therapeutic agent of urinary frequency, urinary incontinence, asthma, chronic obstructive pulmonary disease, rheumatoid arthritis, osteoarthritis, pain, cough, irritable bowel syndrome, emesis, depression, anxiety, manic depressive psychosis or schizophrenia,

[14] a method for the prophylaxis or treatment of urinary frequency, urinary incontinence, asthma, chronic obstructive pulmonary disease, rheumatoid arthritis, osteoarthritis, pain, cough, irritable bowel syndrome, emesis, depression, anxiety, manic depressive psychosis or schizophrenia, which comprises administering an effective amount of (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g] [1,7]naphthyridine and an effective amount of an anti-cholinergic drug in combination to a mammal,

[15] use of (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine and an anti-cholinergic drug for the production of a prophylactic or therapeutic agent of urinary frequency, urinary incontinence, asthma, chronic obstructive pulmonary disease, rheumatoid arthritis, osteoarthritis, pain, cough, irritable bowel syndrome, emesis, depression, anxiety, manic depressive psychosis or schizophrenia,

[16] a commercial package comprising the pharmaceutical agent of the above-mentioned [1] and written matter associated therewith, the written matter stating that the pharmaceutical agent can or should be used for the prophylaxis or treatment of urinary frequency, urinary incontinence, asthma, chronic obstructive pulmonary disease, rheumatoid arthritis, osteoarthritis, pain, cough, irritable bowel syndrome, emesis, depression, anxiety, manic depressive psychosis or schizophrenia.

In addition, compound (Ia) wherein $R^a$ and $R^b$ are bonded to each other to form ring A, which is represented by the formula

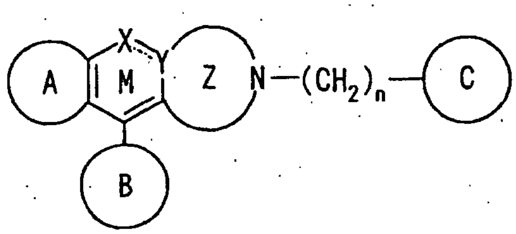

(Ia)

wherein each symbol in the formula is as defined above, is also encompassed in compound (I), and compound (Ia), a salt thereof and a prodrug thereof are also used for the combination drug of the present invention.

In the present specification, compound (I), a salt thereof and a prodrug thereof are also hereinafter generally referred to as compound (T).

**Detailed Description of the Invention**

While the NK-1 receptor antagonist to be used in the present invention is not particularly limited, piperidine derivatives such as CP-122721, GR-205171, HSP-117 and the like, morpholine derivatives such as L-760375, L-758298, MK-869 (L-754030) and the like, piperidine amido derivatives such as NKP-608-C (CGP-60829), SR-140333 and the like, amido derivatives such as R-673, OT-7100, FK-355, R-116301 and the like, perhydroisoindole derivatives such as RPR-100893 and the like, pseudopeptide derivatives such as CI-1021(PD-154075) and the like, as well as LY-303870, SDZ-NKT-343, BIMT-17, GW-597599, L-759274, DA-5018 or salts thereof and the like are exemplified in addition to compound (T). Of these, compound (T) is particularly preferable.

The symbols to be used in the chemical formulas described in the present specification are explained in the following.

**"ring M, X and Y":**

In the above-mentioned formula (I), ring M is a heterocyclic ring having -N=C<, -CO-N< or -CS-N< as the partial structure:
— X ------ Y < . Preferably, ring M has -CO-N< or -N=C< as the partial structure:

— X ------ Y< .

**"$R^a$ and $R^b$":**

In the above-mentioned formula (I), $R^a$ and $R^b$ are bonded to each other to form ring A, or these are the same or different and each represent a hydrogen atom or a substituent on ring M.

The substituents $R^a$ and $R^b$ on ring M include, for example, a halogen atom, an optionally substituted alkyl group, an optionally halogenated alkoxy group, an optionally halogenated alkylthio group, a cycloalkyl group, an aryl group, an acylamino group, an acyloxy group, a hydroxyl group, a nitro group, a cyano group, an amino group, a mono- or di-alkylamino group, a cyclic amino group (e.g., acyclic amino group optionally containing hetero atom(s) of oxygen atom, sulfur atom, etc., in addition to nitrogen atom), an alkylcarbonylamino group, an alkylsulfonylamino group, an alkoxycarbonyl group, a carboxyl group, an alkylcarbonyl group, a carbamoyl group, a mono- or di-alkylcarbamoyl group, an alkylsulfonyl group, an oxo group, etc.

The above-mentioned "halogen atom" includes, for example, fluorine, chlorine, bromine and iodine atoms. Preferably, the halogen atom includes, for example, fluorine, chlorine and bromine atoms.

The "optionally substituted alkyl group" includes, for example, a $C_{1-6}$ alkyl groups (e.g., methyl, ethyl, propyl, isopropyl, butyl, isobutyl, sec-butyl and tert-butyl groups, etc.) optionally having from 1 to 5 substituents selected from a hydroxyl group, a $C_{1-6}$ alkoxy group (e.g., $C_{1-4}$ alkoxy group such as methoxy, ethoxy, propoxy, butoxy, isobutoxy, sec-butoxy, tert-butoxy, etc.), a $C_{1-6}$ alkylthio group (e.g., $C_{1-4}$ alkylthio group such as methylthio, ethylthio, propylthio, butylthio, isobutylthio, sec-butylthio, tert-butylthio, etc.), an amino group, a $C_{1-7}$ acylamino group (e.g. formylamino, acetylamino, propionylamino, butyrylamino, benzoylamino, etc.), an N-alkylamino group, a carboxyl group, a nitro group, a mono- or di-$C_{1-6}$ alkylamino group (e.g., mono- or di-$C_{1-4}$ alkylamino group such as methylamino, ethylamino, propylamino, butylamino, dimethylamino and diethylamino groups, etc.), an optionally substituted N-substituted amino group substituted by one or two homocyclic ring (e.g., mono- or di- $C_{3-8}$ cycloalkylamino groups, for example, cyclopropylamino, cyclobutylamino, cyclohexylamino groups, etc.; $C_{6-10}$ arylamino groups, for example, phenylamino group, etc.), an optionally substituted heterocyclic groups [e.g., 5-membered to 9-membered cyclic amino groups which may have 1 to 3 hetero atoms selected from oxygen atom, sulfur atom and the like in addition to nitrogen atom (e.g., 5-membered or 6-membered non-aromatic cyclic, amino groups, for example, piperidino, 4-methylpiperidino, morpholino, thiomorpholino, piperazinyl, 4-methylpiperazinyl, 4-ethylpiperazinyl, pyrrolidinyl, imidazolydinyl, pyrazolydinyl; 5-membered or 6-membered aromatic cyclic amino groups, for example, pyridyl, pyrazyl, pyrimidinyl, pyridazinyl, pyrrolyl, imidazolyl, pyrazolyl, etc.), aromatic heterocyclic ring groups (e.g., thiophenyl, furanyl, thiazole, isothiazole, oxazole, isoxazole groups, etc.), non-aromatic heterocyclic ring groups (e.g., tetrahydropyridyl, dihydropyridyl, tetrahydropyrazyl, tetrahydropyrimidinyl, tetrahydropyridazinyl, dihydropyranyl, dihydropyrrolyl, dihydroimidazolyl, dihydropyrazolyl, dihydrothiophenyl, dihydrofuranyl, dihydrothiazolyl, dihydroisothiazolyl, dihydroxazolyl, dihydroisoxazolyl, hexahydropyrimidinyl, hexahydropyridazinyl, tetrahydropyranyl, pyrazolidinyl, tetrahydrothiophenyl, tetrahydrofuranyl, tetrahydrothiazolyl, tetrahydroisothiazolyl, tetrahydroxazolyl, tetrahydroisoxazolyl groups, etc.)], an alkylsulfonylamino groups (e.g. $C_{1-4}$ alkylsulfonylamino groups, for example, methylsulfonylamino, ethylsulfonylamino groups, etc.), a $C_{1-6}$ alkylcarbonyloxy group (e.g., $C_{1-4}$ alkyl-carbonyloxy group, for example, acetoxy, ethylcarbonyloxy, propylcarbonyloxy and butylcarbonyloxy groups, etc.) and a halogen atom (e.g., fluorine, chlorine and bromine atoms, etc.), etc.

Preferably, the "optionally substituted alkyl group" includes $C_{1-6}$ alkyl groups optionally substituted by from 1 to 4 or so halogen atoms, especially optionally halogenated $C_{1-4}$ alkyl groups (e.g., $C_{1-4}$ alkyl groups and $C_{1-4}$ alkyl groups substituted by from 1 to 5 (particularly from 1 to 3) or so halogen atoms, etc., such as methyl, chloromethyl, fluoromethyl, difluoromethyl, trichloromethyl, trifluoromethyl, ethyl, 2-bromoethyl, 2,2,2-trichloroethyl, 2,2,2-trifluoroethyl, pentafluoroethyl, propyl, 3,3,3-trifluoropropyl, isopropyl, 1-(trifluoromethyl)ethyl, butyl, 4,4,4-trifluorobutyl, isobutyl, sec-butyl and tert-butyl groups, etc.),

$C_{1-6}$ alkoxy-$C_{1-6}$ alkyl groups (e.g. $C_{1-4}$ alkoxy-$C_{1-4}$ alkyl groups, for example, methoxymethyl, ethoxymethyl, isopropoxymethyl, butoxymethyl, methoxyethyl, ethoxyethyl, etc.),

$C_{1-6}$ alkylthio-$C_{1-6}$ alkyl groups (e.g. $C_{1-4}$ alkylthio-$C_{1-4}$ alkyl groups, for example, methylthiomethyl, ethylthiomethyl, butylthiomethyl, methylthioethyl, ethylthioethyl, etc.), amino-$C_{1-6}$ alkyl groups (e.g., amino-$C_{1-4}$ alkyl groups, for example, aminomethyl, 2-aminoethyl, 2-aminopropyl, 3-aminopropyl, 2-aminobutyl, 3-aminobutyl and 4-aminobutyl groups, etc.),

$C_{1-7}$ acylamino-$C_{1-6}$ alkyl groups (e.g. $C_{1-7}$ acylamino-$C_{1-4}$ alkyl groups, for example, formylaminomethyl, acetylaminomethyl, propionylaminomethyl, formylaminoethyl, acetylaminoethyl, propionylaminoethyl, butylylaminoethyl, benzoylaminomethyl groups, etc.),

mono- or di-$C_{1-6}$ alkylamino-$C_{1-6}$ alkyl groups (e.g. mono- or di-$C_{1-4}$ alkylamino-$C_{1-4}$ alkyl groups, for example, methylaminomethyl, ethylaminomethyl, butylaminomethyl, dimethylaminomethyl, diethylaminomethyl, 2-(N-methylamino) ethyl, 2-(N-ethylamino)ethyl, 2-(N-methylamino)propyl, 3-(N-methylamino)propyl, 3-(N-methylamino)butyl, 4-(N-methylamino)butyl, 2-(N-dimethylamino)ethyl, 2-(N-diethylamino)ethyl groups, etc.) $C_{3-10}$ cycloalkylamino-$C_{1-6}$ alkyl groups (e.g. $C_{3-10}$ cycloalkylamino-$C_{1-4}$ alkyl groups, for example, cyclopropylaminomethyl, cyclobutylaminomethyl, cyclohexylaminomethyl, cyclopropylaminoethyl, cyclobutylaminoethyl, cyclohexylaminoethyl, phenylaminomethyl groups, etc.),

5-membered or 6-membered cyclic amino-$C_{1-6}$ alkyl groups optionally having 1 to 3 hetero atoms selected from oxygen atom and sulfur atom, etc., in addition.to nitrogen atom (e.g. non-aromatic cyclic amino-$C_{1-4}$ alkyl groups, for example, piperidinomethyl, 4-methylpiperidinomethyl, morpholinomethyl, thiomorpholinomethyl, piperazinylmethyl, 4-methylpiperazinylmethyl, piperidinoethyl, morpholinoethyl, piperazinylethyl, etc.; 5-membered or 6-membered aromatic cyclic amino-$C_{1-4}$ alkyl groups, for example, pyridylmethyl, pyrimidinylmethyl, imidazolylmethyl, pyridylethyl, etc.), $C_{1-6}$ alkylsulfonylamino-$C_{1-6}$ alkyl groups (e.g. $C_{1-6}$ alkylsulfonylamino-$C_{1-6}$ alkyl groups, for example, methylsulfonylaminomethyl, ethylsulfonylaminomethyl, methylsulfonylaminoethyl; ethylsulfonylaminoethyl groups, etc.), $C_{1-6}$ alkyl-carbonyloxy-$C_{1-6}$ alkyl groups (e.g. $C_{1-4}$ alkyl-carbonyloxy-$C_{1-4}$, alkyl groups, for example, methylcarbonyloxymethyl, ethylcarbonyloxymethyl, butylcarbonyloxymethyl, methylcarbonyloxyethyl, ethylcarbonyloxyethyl groups, etc.), etc.

The "optionally halogenated alkoxy group" includes, for example, $C_{1-6}$ alkoxy groups or $C_{1-6}$ alkoxy groups substituted by from 1 to 5 or so halogen atoms, etc. Such alkoxy groups or halogenated alkoxy groups include, for example, methoxy, fluoromethoxy, difluoromethoxy, trifluoromethoxy, trichloromethoxy, ethoxy, 2,2,2-trifluoroethoxy, 2,2,2-trichloroethoxy, pentafluoroethoxy, propoxy, isopropoxy, butoxy, 4,4,4-trifluorobutoxy, isobutoxy, sec-butoxy, pentoxy and hexyloxy groups, etc. Preferably, the "optionally halogenated alkoxy group" includes $C_{1-4}$ alkoxy groups or $C_{1-4}$ alkoxy group substituted by from 1 to 3 or so halogen atoms, for example, methoxy, difluoromethoxy, trifluoromethoxy, ethoxy, 2,2,2-trifluoroethoxy, propoxy, isopropoxy, butoxy, 4,4,4-trifluorobutoxy, isobutoxy and sec-butoxy groups, etc.

The "optionally halogenated alkylthio group" includes, for example, $C_{1-6}$ alkylthio groups, and $C_{1-6}$ alkylthio groups having from 1 to 5 or so halogen atoms, etc. Such alkylthio groups and halogenated alkylthio groups include, for example, methylthio, difluoromethylthio, trifluoromethylthio, ethylthio, propylthio, isopropylthio, butylthio, 4,4,4-trifluorobutylthio, pentylthio and hexylthio groups, etc. Preferably, the "optionally halogenated alkylthio group" includes $C_{1-4}$ alkylthio groups, or $C_{1-4}$ alkylthio groups substituted by from 1 to 3 or so halogen atoms, for example, methylthio, difluoromethylthio, trifluoromethylthio, ethylthio, propylthio, isopropylthio, butylthio and 4,4,4-trifluorobutylthio groups, etc.

Furthermore, the "cycloalkyl group" includes $C_{3-10}$ cycloalkyi groups (e.g., cyclopropyl, cyclobutyl, cyclopentyl, cyclohexyl and cyclooctyl groups, etc.) and the like; the "aryl group" includes $C_{6-10}$ aryl groups (e.g., phenyl group, etc.); the "acylamino group" includes, for example, $C_{1-7}$ acylamino groups (e.g., formylamino, acetylamino, propionylamino, butyrylamino and benzoylamino groups, etc.), etc. The "acyloxy group" includes, for example, $C_{1-3}$ acyloxy groups (e.g., formyloxy, acetoxy and propionyloxy groups, etc.), etc. The "mono- or di-alkylamino group" includes, for example, mono- or di-$C_{1-4}$ alkylamino groups (e.g., methylamino, ethylamino, propylamino, dimethylamino groups, etc.), etc. The "cyclic amino group" includes, for example, 5-membered to 9-membered cyclic amino groups optionally having from 1 to 3 hetero atoms, such as oxygen atom, sulfur atom, etc., in addition to nitrogen atom (e.g., pyrrolidino, piperidino, morpholino and thiomorpholino groups, etc.), etc. The "alkylcarbonylamino group" includes, for example, $C_{1-4}$ alkyl-carbonylamino groups (e.g., acetylamino, propionylamino and butyrylamino groups, etc.); the "alkylsulfonylamino group" includes, for example, $C_{1-4}$ alkylsulfonylamino groups (e.g., methylsulfonylamino and ethylsulfonylamino groups, etc.); the "alkoxycarbonyl group". includes, for example, $C_{1-4}$ alkoxy-carbonyl groups (e.g., methoxycarbonyl, ethoxycarbonyl, propoxycarbonyl and butoxycarbonyl groups, etc.); the "alkylcarbonyl group" includes, for example, $C_{1-6}$ alkyl-carbonyl groups (e.g., formyl, methylcarbonyl, ethylcarbonyl and propylcarbonyl groups, etc.); the "mono- or di-alkylcarbamoyl group" includes for example, mono- or di-$C_{1-4}$ alkylcarbamoyl groups (e.g., methylcarbamoyl, ethylcarbamoyl, dimethylcarbamoyl and diethylcarbamoyl groups, etc.); the "alkylsulfonyl group" includes, for example, $C_{1-6}$ alkylsulfonyl groups (e.g., methylsulfonyl, ethylsulfonyl and propylsulfonyl groups, etc.), etc.

**"ring A and ring B":**

In the above-mentioned formula (I), ring A and ring B each represent a homocyclic ring or heterocyclic ring optionally having substituents, and at least one of these is a heterocyclic ring optionally having substituents.

The "homocyclic ring or heterocyclic ring" includes, for example, (i) an aromatic heterocyclic ring or non-aromatic heterocyclic ring having one or two kinds of hetero atoms selected from nitrogen, sulfur and oxygen atoms, preferably from 1 to 3 such hetero atoms, in addition to carbon atoms, or (ii) a cyclic hydrocarbon (homocyclic ring) consisting of carbon atoms, etc.

The "aromatic heterocyclic ring" includes, for example, 5-membered or 6-membered aromatic heterocyclic rings having 1 to 3 hetero atoms selected from nitrogen, oxygen and sulfur atoms, in addition to carbon atoms (e.g., pyridine, pyrazine, pyrimidine, pyridazine, pyrrole, imidazole, pyrazole, triazole, thiophene, furan, thiazole, isothiazole, oxazole and isoxazole rings, etc.), etc. Preferably, the aromatic heterocyclic ring includes, for example, pyridine, pyrazine and thiophene rings, etc., as well as, for example, pyrrole and thiazole rings, etc. Especially preferred are (i) 6-membered, nitrogen-containing heterocyclic rings having one or two nitrogen atoms in addition to carbon atoms (e. g., pyridine and pyrazine rings, etc.) or (ii) 5-membered aromatic heterocyclic rings having one sulfur atom in addition

to carbon atoms (e.g., thiophene ring, etc.), etc.

The "non-aromatic heterocyclic ring" includes, for example, 5-membered to 9-membered, non-aromatic heterocyclic rings, preferably 5-membered or 6-membered, non-aromatic heterocyclic rings, having from 1 to 3 hetero atoms selected from nitrogen, oxygen and sulfur atoms in addition to carbon atoms, etc.

For example, ring A includes tetrahydropyridine, dihydropyridine, tetrahydropyrazine, tetrahydropyrimidine, tetrahydropyridazine, dihydropyran, dihydropyrrole, dihydroimidazole, dihydropyrazole, dihydrothiophene, dihydrofuran, dihydrothiazole, dihydroisothiazole, dihydroxazole and dihydroisoxazole rings, etc.; and ring B includes, in addition to the above mentioned rings, piperidine, piperazine, hexahydropyrimidine, hexahydropyridazine, tetrahydropyran, morpholine, pyrrolidine, imidazolidine, pyrazolidine, tetrahydrothiophene, tetrahydrofuran, tetrahydrothiazole, tetrahydroisothiazole, tetrahydroxazole and tetrahydroisoxazole rings, etc. Preferably, ring A includes, for example, 6-membered, non-aromatic heterocyclic rings having one or two nitrogen atoms in addition to carbon atoms (e.g., tetrahydropyridine, tetrahydropyrimidine and tetrahydropyridazine rings, etc.), etc., and is especially commonly used a tetrahydropyridine ring, etc. Preferably, ring B includes, for example, 6-membered, non-aromatic heterocyclic rings having one or two nitrogen atoms in addition to carbon atoms (e.g., piperidine and piperazine rings, etc.), etc., and is especially commonly used a piperazine ring, etc.

The "cyclic hydrocarbon (homocyclic ring)" includes, for example, 3-membered to 10-membered (for example, 5-membered to 9-membered) cyclic hydrocarbon, preferably 5-membered or 6-membered cyclic hydrocarbon, etc. For example, ring A includes benzene, $C_{3-10}$ cycloalkenes (e.g., cyclobutene, cyclopentene, cyclohexene, cycloheptene, cyclooctene, etc.), etc. The cycloalkenes are preferably $C_{5-6}$ cycloalkenes (e.g., cyclopentene, cyclohexene, etc.), etc. ring B includes, in addition to the above-mentioned rings, $C_{3-10}$ cycloalkanes (e.g., cyclobutane, cyclopentane, cyclohexane, cycloheptane, cyclooctane, etc.), etc. The cycloalkanes are preferably $C_{5-6}$ cycloalkanes (e.g., cyclohexane, cyclopentane, etc.), etc. Preferably, ring A includes, for example, 6-membered homocyclic rings such as benzene and cyclohexene rings, etc. Especially preferred are a benzene ring, etc. ring B preferably includes, for example, 6-membered homocyclic rings such as benzene and cyclohexane rings, etc. Especially preferred is a benzene ring.

At least one of ring A and ring B is a heterocyclic ring optionally having substituents. Both of ring A and ring B may be heterocyclic rings optionally having substituents. Preferably, one of ring A and ring B is 1) an aromatic ring optionally having substituents and the other is 2) a heterocyclic ring (preferably, aromatic heterocyclic ring) optionally having substituents.

The above-mentioned 1) "aromatic ring" includes, for example, (i) the above-mentioned "aromatic heterocyclic rings", namely, optionally substituted, 5-membered or 6-membered, aromatic heterocyclic rings havingone or two kind of hetero atoms selected from nitrogen, sulfur and oxygen atoms, preferably from 1 to 3 such hetero atoms, in addition to carbon atoms (e.g., pyridine, pyrazine, pyrimidine, pyridazine, pyrrole, imidazole, pyrazole, triazole, thiophene, furan, thiazole, isothiazole, oxazole and isoxazole rings, etc.), and (ii) benzene rings optionally having substituents.

As the substituents the "aromatic ring" may have in the above-mentioned 1), for example, referred to are the same substituents as those for ring A and ring B which are mentioned hereinunder. The "aromatic heterocyclic ring" of the "aromatic heterocyclic ring optionally having substituents" in the above-mentioned 2) includes, for example, the same aromatic heterocyclic rings as those in the above-mentioned "5-membered or 6-membered, aromatic heterocyclic ring". As the substituents the "aromatic heterocyclic ring optionally having substituents" in the above-mentioned 2) may have, for example, referred to are the same substituents as those for ring A and ring B which are mentioned hereinunder. The "5-membered or 6-membered, aromatic heterocyclic ring" preferably includes the same heterocyclic rings and the like as those in the above-mentioned "aromatic heterocyclic ring".

More preferably, one of ring A and ring B is an aromatic heterocyclic ring (e.g., a 5-membered or 6-membered aromatic heterocyclic ring) optionally having substituents and the other is a benzene ring optionally having substituents.

The substituents for the "homocyclic ring or heterocyclic ring", "aromatic heterocyclic ring", "non-aromatic heterocyclic ring", "cyclic hydrocarbon", "aromatic ring" and "benzene ring", which optionally have substituents and are to be represented by ring A and ring B, include, for example, a halogen atom, an optionally substituted alkyl group, an optionally halogenated alkoxy group, an optionally halogenated alkylthio group, an aryl group, an acylamino group, an acyloxy group, a hydroxyl group, a nitro group, a cyano group, an amino group, a mono- or di-alkylamino group, a cyclic amino group (e.g., a cyclic amino group optionally having hetero atom selected from oxygen atom, sulfur atom, etc., in addition to nitrogen atom), an alkylcarbonylamino group, an alkylsulfonylamino group, an alkoxycarbonyl group, a carboxyl group, an alkylcarbonyl group, a carbamoyl group, a mono- or di-alkylcarbamoyl group, an alkylsulfonyl group, an oxo group, etc.

The "halogen atom", which ring A and ring B may have, includes, for example, fluorine, chlorine, bromine and iodine atoms. Preferably, the halogen atom includes, for example, fluorine, chlorine and bromine atoms (especially, fluorine and chlorine atoms, etc.).

The "optionally substituted alkyl group", which ring A and ring B may have, includes, for example, $C_{1-6}$ alkyl groups (e.g., methyl, ethyl, propyl, isopropyl, butyl, isobutyl, sec-butyl and tert-butyl groups, etc.) and the like optionally having from 1 to 5 substituents selected from a hydroxyl group, an amino group, a carboxyl group, a nitro group, a

mono- or di-$C_{1-6}$ alkylamino group (e.g., methylamino, ethylamino, dimethylamino and diethylamino groups, etc.), a $C_{1-6}$ alkyl-carbonyloxy group (e.g., acetoxy and ethylcarbonyloxy groups, etc.) and a halogen atom (e.g., fluorine, chlorine and bromine atoms, etc.), etc. Especially preferred are optionally halogenated alkyl groups, for example, $C_{1-6}$ alkyl groups, and $C_{1-6}$ alkyl groups substituted by from 1 to 4 or so halogen atoms, etc. Such alkyl groups and halogenated alkyl groups include, for example, methyl, chloromethyl, fluoromethyl, difluoromethyl, trichloromethyl, trifluoromethyl, ethyl, 2-bromoethyl, 2,2,2-trichloroethyl, 2,2,2-trifluoroethyl, pentafluoroethyl, propyl, 3,3,3-trifluoropropyl, isopropyl, 2-trifluoromethylethyl, butyl, 4,4,4-trifluorobutyl, isobutyl, sec-butyl, tert-butyl, pentyl, isopentyl, neopentyl, 5,5, 5-trif luoropentyl, 4-trif luoromethylbutyl, hexyl1 6,6,6-trifluorohexyl and 5-trif luoromethylpentyl. groups, etc.

More preferably, the "optionally substituted alkyl group". includes optionally halogenated $C_{1-4}$ alkyl groups, for example, $C_{1-4}$ alkyl groups and $C_{1-4}$ alkyl groups substituted by from 1 to 3 or so halogen atoms, etc., such as methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, 2-trifluoromethylethyl, butyl, 4,4,4-trifluorobutyl, isobutyl, sec-butyl and tert-butyl groups, etc.

The "optionally halogenated alkoxy group", which ring A and ring B may have, includes, for example, $C_{1-6}$ alkoxy groups or $C_{1-6}$ alkoxy groups substituted by from 1 to 5 or so halogen atoms such as those mentioned hereinabove, etc. Such alkoxy groups or halogenated alkoxy groups include, for example, methoxy, difluoromethoxy, trifluoromethoxy, trichloromethoxy, ethoxy, 2,2,2-trifluoroethoxy, 2,2,2-trichloroethoxy, pentafluoroethoxy, propoxy, isopropoxy, butoxy, 4,4,4-trifluorobutoxy, isobutoxy, sec-butoxy; pentoxy and hexyloxy groups, etc. Preferably, the "optionally halogenated alkoxy group" includes $C_{1-4}$ alkoxy groups or $C_{1-4}$ alkoxy group substituted by from 1 to 3 or so halogen atoms, for example, methoxy, difluoromethoxy, trifluoromethoxy, ethoxy, 2,2,2-trifluoroethoxy, propoxy, isopropoxy, butoxy, 4,4,4-trifluorobutoxy, isobutoxy and sec-butoxy groups, etc.

The "optionally halogenated alkylthio group", which ring A and ring B may have, includes, for example, $C_{1-6}$ alkylthio groups, and $C_{1-6}$ alkylthio groups having from 1 to 5 or so halogen atoms such as those mentioned hereinabove, etc. Such alkylthio groups and halogenated alkylthio groups include, for example, methylthio, difluoromethylthio, trifluoromethylthio, ethylthio, propylthio, isopropylthio, butylthio, 4,4,4-trifluorobutylthio, pentylthio and hexylthio groups, etc. Preferably, the "optionally halogenated alkylthio group" includes $C_{1-4}$ alkylthio groups, or $C_{1-4}$ alkylthio groups substituted by from 1 to 3 or so halogen atoms, for example, methylthio,. difluoromethylthio, trifluoromethylthio, ethylthio, propylthio, isopropylthio, butylthio and 4,4,4-trifluorobutylthio groups, etc.

The aryl group as the substituent includes $C_{6-10}$ aryl groups (e.g., phenyl group, etc.); the acylamino group includes, for example, $C_{1-7}$ acylamino groups (e.g., formylamino, acetylamino, propionylamino, butyrylamino and benzoylamino groups, etc.), etc. The acyloxy group includes, for example, $C_{1-3}$ acyloxy groups (e.g., formyloxy, acetoxy and propionyloxy groups, etc.), etc. The mono- or di-alkylamino group includes, for example, mono- or di-$C_{1-4}$ alkylamino groups (e.g., methylamino, ethylamino, propylamino, dimethylamino and diethylamino groups, etc.), etc. The cyclic amino group includes, for example, 5-membered to 9-membered cyclic amino groups optionally having from 1 to 3 hetero atoms, such as oxygen atom, sulfur atom, etc., in addition to nitrogen atom (e.g., pyrrolidino, piperidino and morpholino groups, etc.), etc. The alkylcarbonylamino group includes, for example, $C_{1-4}$ alkyl-carbonylamino groups (e.g., acetylamino, propionylamino and butyrylamino groups, etc.); the alkylsulfonylamino group includes, for example, $C_{1-4}$ alkylsulfonylamino groups (e.g., methylsulfonylamino and ethylsulfonylamino groups, etc.); the alkoxycarbonyl group includes, for example, $C_{1-4}$ alkoxy-carbonyl groups (e.g., methoxycarbonyl,.ethoxycarbonyl, propoxycarbonyl and butoxycarbonyl groups, etc.); the alkylcarbonyl group includes, for example, $C_{1-6}$ alkyl-carbonyl groups (e.g., formyl, methylcarbonyl, ethylcarbonyl and propylcarbonyl groups., etc.); the mono- or di-alkylcarbamoyl group includes, for example, mono- or di-$C_{1-4}$ alkylcarbamoyl groups (e.g., methylcarbamoyl, ethylcarbamoyl, dimethylcarbamoyl and diethylcarbamoyl groups, etc.); the alkylsulfonyl group includes, for example, $C_{1-6}$ alkylsulfonyl groups (e.g., methylsulfonyl, ethylsulfonyl and propylsulfonyl groups, etc.), etc.

The terminology "optionally halogenated" as referred to herein means that the number of halogen atoms is from 1 to 5, preferably from 1 to 3 or so.

Preferred substituents which ring A and ring B may have include a halogen atom, an optionally halogenated $C_{1-4}$ alkyl group, an optionally halogenated $C_{1-4}$ alkoxy group, an optionally halogenated $C_{1-4}$ alkylthio group, a $C_{1-3}$ acyloxy group, a hydroxyl group, an amino group, a mono- or di-$C_{1-4}$ alkylamino group, a carboxyl group, a $C_{1-4}$ alkoxycarbonyl group, an oxo group, etc.

More preferred substituents which ring A and ring B may have include a halogen atom, an optionally halogenated $C_{1-4}$ alkyl group, an optionally halogenated $C_{1-4}$ alkoxy group, a hydroxyl group, an amino group, a mono- or di-$C_{1-4}$ alkylamino group, a $C_{1-3}$ acyloxy group, an oxo group, etc. Especially preferred are a halogen atom, an optionally halogenated $C_{1-4}$ alkyl group, an optionally halogenated $C_{1-4}$ alkoxy group, etc.

The substituents for ring A and ring B may be at any substitutable position on the ring. If the rings are substituted by two or more substituents, the substituents may be the same or different. The number of the substituents may be from 1 to 4 or so, preferably from 1 to 3 or so.

If the ring A and/or the ring B has(have) nitrogen atom(s), the ring may form a quaternary ammonium salt.

For example, it may form a salt with anion(s) such as halide ion(s) (e.g., $Cl^-$, $Br^-$, $I^-$, etc.), sulfate ion, hydroxy ion, and the like.

**"ring A":**

Preferred homocyclic rings for ring A are homocyclic rings optionally having substituents, which are composed of carbon atoms, for example, including those of the formula (A-1):

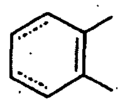 , 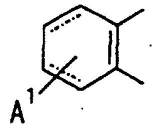 (A-1)

wherein ------ indicates a single bond or a double bond and the same shall apply hereinunder; and $A^1$ represents a halogen atom (e.g., fluorine and chlorine atoms, etc.), an optionally halogenated $C_{1-4}$alkyl group (e.g., methyl, isopropyl, trifluoromethyl, trichloromethyl, ethyl, 2,2,2-trifluoroethyl and pentafluoroethyl groups, etc.), or an optionally. halogenated $C_{1-4}$ alkoxy group (e.g., methoxy, trifluoromethoxy, trichloromethoxy, ethoxy, 2,2,2-trifluorpethoxy and pentafluoroethoxy groups, etc.); or those of the formula (A-2) and the like:

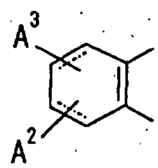 (A-2)

wherein $A^2$ and $A^3$ are the same or different and each represent a halogen atom (e.g., fluorine and chlorine atoms, etc.), an optionally halogenated $C_{1-4}$ alkyl group (e.g., methyl, isopropyl, trifluoromethyl, trichloromethyl, ethyl, 2,2,2-trifluoroethyl and pentafluoroethyl groups, etc.), or an optionally halogenated $C_{1-4}$ alkoxy group (e.g., methoxy, trifluoromethoxy, trichloromethoxy, ethoxy, 2,2,2-trifluroroethoxy and pentafluoroethoxy groups, etc.). More preferred homocyclic rings include, for example, homocyclic rings (especially benzene ring) of the formula (A-3) and the like:

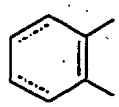 or 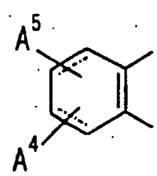 (A-3)

wherein $A^4$ and $A^5$ are the same or different and each represent a halogen atom (e.g., fluorine and chlorine atoms, etc.), or an optionally halogenated $C_{1-4}$ alkyl group (e.g., methyl, trifluoromethyl, trichloromethyl, ethyl, 2,2,2-trifluoroethyl, pentafluoroethyl and isopropyl groups, etc.).

Also, as the homocyclic ring, preferred is a benzene ring optionally having substituents, which is represented by the formula (A-4):

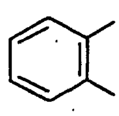 , 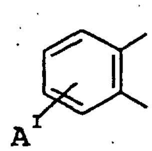 or 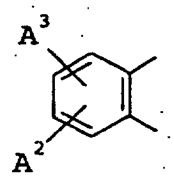

particularly,

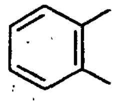 or 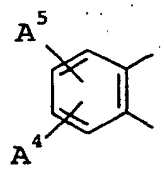 (A-4)

wherein the symbols are as defined above.

Of the homocyclic rings of the above-mentioned formulas, especially preferred are those as substituted by. the following substituent(s):

(1) Homocyclic rings wherein $A^1$ is a halogen atom (e.g., fluorine and chlorine atoms, etc.), or an optionally halogenated $C_{1-4}$ alkyl group (e.g., methyl, triflupromethyl, ethyl and isopropyl groups, etc.).
(2) Homocyclic rings wherein $A^2$ and $A^3$ are the same or different and each represent an optionally halogenated $C_{1-4}$ alkyl group (e.g., methyl, trifluoromethyl, ethyl and isopropyl groups, etc.), or an optionally halogenated $C_{1-4}$ alkoxy group (e.g., methoxy, trifluoromethoxy and ethoxy groups, etc.).
(3) Homocyclic rings wherein $A^4$ and $A^5$ are the same or different and each represent a $C_{1-4}$ alkyl group (e.g., methyl, ethyl and isopropyl groups, etc.).
(4) $A^1$ is a halogen atom (e.g., fluorine and chlorine atoms, etc.).
(5) Homocyclic rings wherein $A^2$ and $A^3$ are the same or different and each represent a $C_{1-4}$ alkoxy group (e.g., methoxy and ethoxy groups, etc.).

Preferred aromatic heterocyclic or non-aromatic heterocyclic rings for ring A are 5-membered or 6-membered, aromatic heterocyclic or non-aromatic heterocyclic rings including, for example, pyridine, pyrazine, thiophene, tetrahydropyridine, pyrrole and thiazole rings, etc. Concretely, for example, preferred are heterocyclic rings of the formula (A-5):

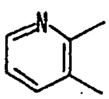 , 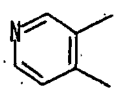 , 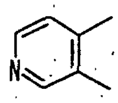 , 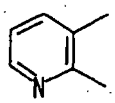 , 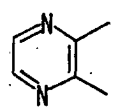 , 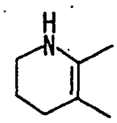

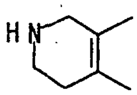 , 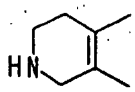 , 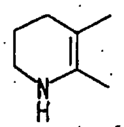 , 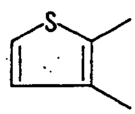 , 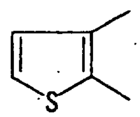 , 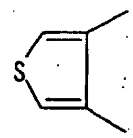

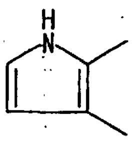 , 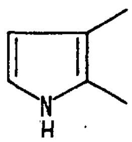 , 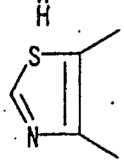 , 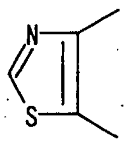 (A-5)

As preferable examples of the aromatic or non-aromatic heterocyclic ring optionally having substituents, mentioned are pyridine, pyrazine, thiophene, tetrahydropyridine, pyrrole and thiazole rings, etc. optionally having one or two substituents selected from an oxo group, an optionally substituted alkyl group (this has the same meaning as the substituent which ring A and ring B may have), a $C_{6-10}$ aryl group (e.g., phenyl group, etc.) and a halogen atom (e.g., fluorine, chlorine and bromine atoms, etc.). Concretely, for example, preferred are aromatic or non-aromatic heterocyclic rings of the formula (A-6):

(i) 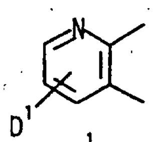, 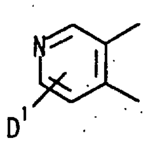, 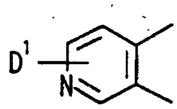, 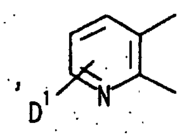, 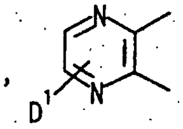 ;

(ii) 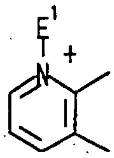, 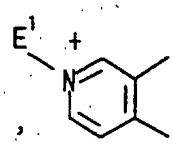, 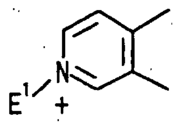, 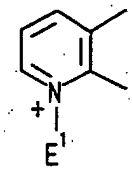 ;

(iii) 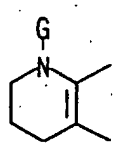, 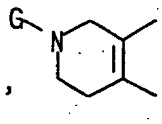, 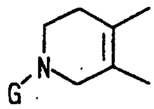, 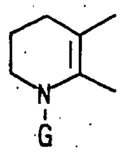 ;

(iv) 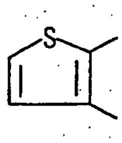, 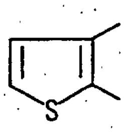, 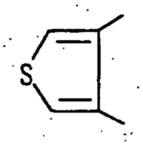

(v) 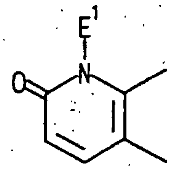

(vi) 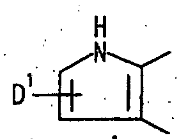, 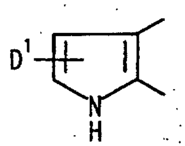 ;

(vii) 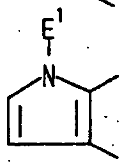, 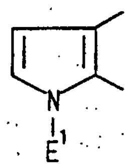

or

(viii) 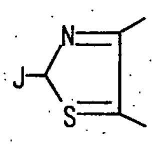, 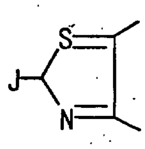 (A-6)

wherein $D^1$ represents a hydrogen atom, a halogen atom (e.g., fluorine, chlorine and bromine atoms, etc.); $E^1$ represents a $C_{1-4}$ alkyl group (e.g., methyl, ethyl, propyl and isopropyl groups, etc.) and the like; the compounds having the partial structure of (ii) form quaternary ammonium salts along with a halide ion (e.g., $Cl^-$, $Br^-$, $I^-$, etc.), a sulfate ion, a hydroxy ion or the like; G represents a hydrogen atom or a $C_{1-4}$ alkyl group (e.g., methyl, ethyl, propyl and isopropyl groups, etc.); J represents a hydrogen atom, a $C_{1-4}$ alkyl group (e.g., methyl, ethyl, propyl and isopropyl groups, etc.)

or a $C_{6-10}$ aryl group (e.g., phenyl group, etc.).

Ring A is preferably a 5-membered or a 6-membered, nitrogen-containing heterocyclic ring, for example, (i) a 6-membered, aromatic, nitrogen-containing heterocyclic ring having one or two nitrogen atoms in addition to carbon atoms (e.g., pyridine and pyrazine rings, etc.), (ii) a 6-membered, non-aromatic heterocyclic ring having one or two nitrogen atoms in addition to carbon atoms (e.g., tetrahydropyridine, tetrahydropyrimidine and tetrahydropyridazine rings, etc.), or the like. Especially preferably, ring A is an aromatic, nitrogen-containing heterocyclic ring, particularly, a pyridine ring or the like.

**"ring B":**

Preferred homocyclic ring for ring B is a homocyclic ring optionally having substituents, which consists of carbon atoms, for example, including those of the formula (B-1):

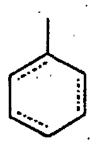
,
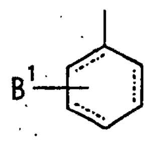
(B-1)

wherein $B^1$ represents a halogen atom, a $C_{1-4}$ alkyl group optionally substituted by hydroxy or optionally halogenated, an optionally halogenated $C_{1-4}$ alkoxy group, a $C_{1-6}$ alkyl-carbonyl group or a carboxyl group;
those of the formula (B-2):

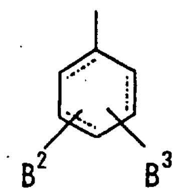
(B-2)

wherein $B^2$ and B3 are the same or different and each represent a halogen atom, an optionally halogenated $C_{1-4}$ alkyl group or an optionally halogenated $C_{1-4}$ alkoxy group; and those of the formula (B-3) like:

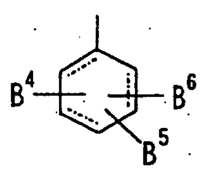
(B-3)

wherein $B^4$, $B^5$ and $B^6$ are the same or different and each represent a halogen atom, an optionally halogenated $C_{1-4}$ alkyl group or an optionally halogenated $C_{1-4}$ alkoxy group.

More preferred are homocyclic rings of the formula (B-4) and the like:

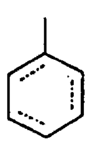
,
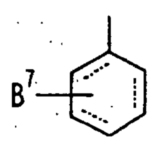
or
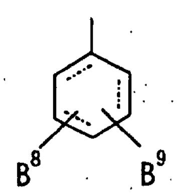
(B-4)

wherein $B^7$, and $B^9$ are the same or different and each represent a halogen atom, an optionally halogenated $C_{1-4}$ alkyl group or an optionally halogenated $C_{1-4}$ alkoxy group.

Even more preferred are homocyclic rings of the formula (B-5):

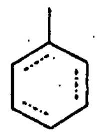 or 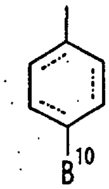 (B-5)

wherein $B^{10}$ represents, a halogen atom, a $C_{1-4}$ alkyl group optionally substituted by hydroxy or optionally halogenated, an optionally halogenated $C_{1-4}$ alkoxy group, a $C_{1-6}$ alkyl-carbonyl group or a carboxyl group.

In the above-mentioned formulas, the halogen atom for any of $B^1$ to $B^{10}$ includes, for example, fluorine, chlorine and bromine atoms, etc.; the optionally halogenated $C_{1-4}$ alkyl group includes, for example, methyl, trifluoromethyl, trichloromethyl, ethyl, 2,2,2-trifluoroethyl, 2,2,2-trichloroethyl, 1,1,2,2-tetrafluoroethyl, pentafluoroethyl, propyl, 2,2,3,3-tetrafluoropropyl and isopropyl groups, etc.; and the optionally halogenated $C_{1-4}$ alkoxy group includes, for example, methoxy, trifluoromethoxy,trichloromethoxy, ethoxy, 2,2,2-trifluoroethoxy, 2,2,2-trichloroethoxy, 1,1,2,2-tetrafluoroethoxy, pentafluoroethoxy, propoxy, 2,2,3,3-tetrafluoropropoxy and isopropoxy groups, etc.

In the above-mentioned formulas, the $C_{1-6}$ alkyl-carbonyl group represented by $B^1$ or $B^{10}$ includes, for example, formyl, acetyl and the like.

Ring B is also preferably a benzene ring optionally having substituents, which preferably includes, for example, benzene rings of the formula (B-6):

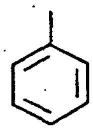 , 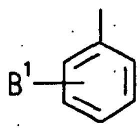 , 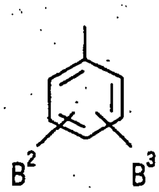 or 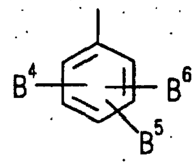 (B-6)

More preferred are benzene rings of the formula (B-7):

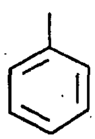 , 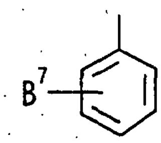 or 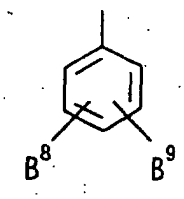 (B-7)

Especially preferred are benzene rings of the formula (B-8) and the like:

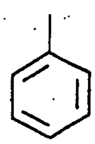 or 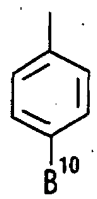 (B-8)

In these formulas, the symbols are as defined above.

Of the substituents in the above-mentioned formulas, for example, especially preferred are the following:

(1) $B^1$, $B^2$, $B^3$, $B^4$, $B^5$ and $B^6$ are the same or different and each represent a halogen atom (e.g., fluorine and chlorine atoms, etc.) or an optionally halogenated $C_{1-4}$ alkyl group (e.g., methyl, trifluoromethyl, ethyl and isopropyl groups, etc.).
(2) $B^1$, $B^2$, $B^3$, $B^4$, $B^5$ and $B^6$ are the same or different and each represent an optionally halogenated $C_{1-4}$ alkoxy group (e.g., methoxy, trifluoromethoxy and ethoxy groups, etc.).
(3) $B^7$, $B^8$ and $B^9$ represent halogen atoms (e.g., fluorine and chlorine atoms, etc.).

(4) $B^{10}$ represents a fluorine atom.
(5) $B^{10}$ represents a $C_{1-4}$ alkyl group (e.g., methyl group, etc.).
(6) $B^1$ or $B^{10}$ represents a $C_{1-6}$ alkyl group which may be substituted by hydroxy (e.g., hydroxymethyl, etc.), a $C_{1-6}$ alkyl-carbonyl group (e.g., formyl, acetyl, etc.), a carboxyl group and the like.

More preferred benzene rings optionally having substituents are phenyl groups of the following formula (B-9):

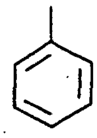 , 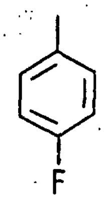 or 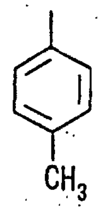 (B-9)

As preferred examples of aromaticheterocyclic rings or non-aromatic heterocyclic rings for ring B, mentioned are 5-membered or 6-membered aromatic heterocyclic rings or non-aromatic heterocyclic rings such as pyridine, thiophene and piperidine rings, etc. These rings may optionally be substituted by substituents such as those mentioned hereinabove as preferred substituents for ring A.
Where ring B is an aromatic heterocyclic ring or a non-aromatic heterocyclic ring, it especially preferably includes, for example, heterocyclic rings of the formula (B-10) and the like:

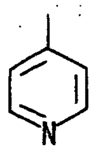 , 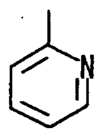 or 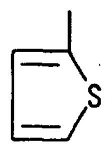 (B-10)

where one or both of ring A and ring B is/are heterocyclic ring(s), the ring(s) is/are also preferably unsubstituted one (s).

**Combination of ring A and ring B**

Preferred combination of ring A and ring B (1) is as follows:

(1) One of ring A and ring B is a 5-membered or 6-membered heterocyclic ring having one or two hetero atoms selected from nitrogen and sulfur atoms in addition to carbon atoms (e.g., pyridine, pyrazine, thiophene, tetrahydropyridine, piperidine and piperazine rings, etc.) which may be optionally substituted by $C_{1-4}$ alkyl group(s) (e.g., methyl, ethyl and isopropyl groups, etc.).
The other of ring A and ring B is a benzene ring optionally substituted by from 1 to 3 substituents selected from a halogen atom (e.g., fluorine, chlorine and bromine atoms, etc.), an optionally halogenated $C_{1-4}$ alkyl group (e.g., methyl, trifluoromethyl, trichloromethyl, ethyl, 2,2,2-trifluoroethyl, pentafluoroethyl, 2,2,2-trichloroethyl, propyl and isopropyl groups, etc.) and an optionally halogenated $C_{1-4}$ alkoxy group (e.g., methoxy, trifluoromethoxy, trichloromethoxy, ethoxy, 2,2,2-trifluoroethoxy, pentafluoroethoxy, 2,2,2-trichloroethoxy, propoxy and isopropoxy groups, etc.).
More preferred combination of ring A and ring B (2) is as follows:
(2) One of ring A and ring B is a 5-membered or 6-membered aromatic heterocyclic ring having one or two hetero atoms selected from nitrogen and sulfur atoms in addition to carbon atoms (e.g., pyridine, pyrazine and thiophene rings, etc.).

The other of ring A and ring B is a benzene ring optionally substituted by from 1 to 3 substituents selected from a halogen atom (e.g., fluorine, chlorine and bromine atoms, etc.), an optionally halogenated $C_{1-4}$ alkyl group (e.g., methyl, trifluoromethyl, trichloromethyl, ethyl, 2,2,2-trifluoroethyl, pentafluoroethyl, 2,2,2-trichloroethyl, propyl and isopropyl groups, etc.) and an optionally halogenated $C_{1-4}$ alkoxy group (e.g.', methoxy, trifluoromethoxy, trichloromethoxy, ethoxy, 2,2,2-trifluoroethoxy, pentafluoroethoxy, 2,2,2-trichloroethoxy, propoxy and isopropoxy groups, etc .).
Especially preferably, ring A is an aromatic heterocyclic ring optionally having substituents such as those mentioned above (e.g., 5-membered or 6-membered aromatic heterocyclic ring, especially pyridine ring, etc.), while ring B is a benzene ring optionally having substituents.

**"ring C":**

In the above-mentioned formulas, ring C represents a homocyclic ring optionally having substituents or a heterocyclic ring optionally having substituents. The homocyclic ring or the heterocyclic ring may have from 1 to 5 or so, preferably from 1 to 3 or so substituents, which may be the same or different. The substituents may be positioned at any position of the homocyclic ring or heterocyclic ring.

The homocyclic ring includes "cyclic hydrocarbon (homocyclic ring)" such as those as referred to hereinabove for "ring A and ring B", for example, from 3-membered to 10-membered cyclic hydrocarbon such as benzene, $C_{3-10}$ cycloalkenes (e.g., cyclobutene, cyclopentene, cyclohexene, cycloheptene, cyclooctene, etc.), $C_{3-10}$ cycloalkanes (e.g., cyclobutane, cyclopentane, cyclohexane, cycloheptane, cyclooctane, etc.), etc., preferably 5-membered or 6-membered cyclic hydrocarbon and the like. Of these, preferred are 6-membered homocyclic rings, such as benzene, cyclohexene and cyclohexane rings, etc. Especially preferred is benzene ring.

The substituents for the homocyclic rings such as the above-mentioned benzene ring include, for example, a halogen atom (e.g., fluorine, chlorine, bromine and iodine atoms), an optionally halogenated $C_{1-10}$ alkyl group (e.g., methyl, chloromethyl, difluoromethyl trichloromethyl, trifluoromethyl, ethyl, 2-bromoethyl, 2,2,2-trifluoroethyl, perfluoroethyl, propyl, isopropyl, 3,3,3-trifluoropropyl, butyl, isobutyl, tert-butyl, perfluorobutyl, pentyl, hexyl, octyl and decyl groups, etc.), an amino-substituted $C_{1-4}$ alkyl group (e.g., aminomethyl and 2-aminoethyl groups, etc.), a mono- or di-$C_{1-4}$ alkylamino-substituted $C_{1-4}$ alkyl group (e.g., methylaminomethyl, dimethylaminomethyl, 2-methylaminoethyl and 2-dimethylaminoethyl groups, etc.), a carboxyl-substituted $C_{1-4}$ alkyl group (e.g., carboxymethyl and carboxyethyl groups, etc.), a $C_{1-4}$ alkoxy-carbonyl-substituted $C_{1-4}$ alkyl group (e.g., methoxycarbonylethyl and ethoxycarbonylethyl groups, etc.), a hydroxy-substituted $C_{1-4}$ alkyl group (e.g., hydroxymethyl and hydroxyethyl groups, etc.), a $C_{1-4}$ alkoxy-carbonyl-substituted $C_{1-4}$ alkyl group (e.g.,methoxymethyl, methoxyethyl and ethoxyethyl groups, etc.), a $C_{3-10}$ cycloalkyl group (e.g., cyclopropyl, cyclobutyl, cyclopentyl, cyclohexyl, cycloheptyl and cyclooctyl groups, etc.), a nitro group, a cyano group, a hydroxyl group, an optionally halogenated $C_{1-10}$ alkoxy group (e.g., methoxy, difluoromethoxy, trichloromethoxy, trifluoromethoxy, ethoxy, 2,2,2-trifluoroethoxy, perfluoroethoxy, propoxy, isopropoxy, butoxy, isobutoxy, tert-butoxy, perfluorobutoxy, pentyloxy, hexyloxy, nonyloxy, octyloxy and decyloxy groups, etc.), an optionally halogenated $C_{1-4}$ alkylthio group (e.g., methylthio, difluoromethylthio, trifluoromethylthio, ethylthio, propylthio, isopropylthio and butylthio groups, etc.), an amino group, a mono- or di-$C_{1-4}$ alkylamino group (e.g., methylamino, ethylamino, propylamino, dimethylamino and diethylamino groups, etc.), a cyclic amino group (e.g., a 5-membered to 9-membered cyclic amino group optionally having from. 1 to 3 hetero atoms such as oxygen and sulfur atoms, etc., in addition to nitrogen atoms, concretely for example, pyrrolidino, piperidino and morpholino groups, etc.), a $C_{1-4}$ alkyl-carbonylamino group (e.g., acetylamino, propionylamino and butyrylamino, etc.), an aminocarbonyloxy group, a mono- or di-$C_{1-4}$ alkylaminocarbonyloxy group (e.g., methylaminocarbonyloxy, ethylaminocarbonyloxy, dimethylaminocarbonyloxy and diethylaminocarbonyloxy groups, etc.), a $C_{1-4}$ alkylsulfonylamino group (e.g., methylsulfonylamino, ethylsulfonylamino and propylsulfonylamino groups, etc.), a $C_{1-4}$ alkoxy-carbonyl group (e.g., methoxycarbonyl, ethoxycarbonyl, propoxycarbonyl, butoxycarbonyl and isobutoxycarbonyl groups, etc.), an aralkyloxycarbonyl group (e.g., $C_{7-19}$ aralkyloxycarbonyl group such as phenyl-$C_{1-4}$ alkyloxy-carbonyl (e.g., benzyloxycarbonyl group, etc.), etc.), a carboxyl group, a $C_{1-6}$ alkyl-carbonyl group (e.g., methylcarbonyl, ethylcarbonyl and butylcarbonyl groups, etc.), a $C_{3-6}$ cycloalkyl-carbonyl group (e.g., cyclohexylcarbonyl group, etc.), a carbamoyl group, a mono- or di-$C_{1-4}$ alkylcarbamoyl group (e.g., methylcarbamoyl, ethylcarbamoyl, propylcarbamoyl, butylcarbamoyl, diethylcarbamoyl and dibutylcarbamoyl groups, etc.), a $C_{1-6}$ alkylsulfonyl group (e.g., methylsulfonyl, ethylsulfonyl and propylsulfonyl groups, etc.), etc.

The monocyclic ring as ring C may optionally be substituted, for example, by one 5-membered or 6-membered, aromatic monocyclic heterocyclic group (e.g., furyl, thienyl, oxazolyl, isoxazolyl, thiazolyl, isothiazolyl, imidazolyl, pyrazolyl,-1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl and triazinyl groups, etc.), etc., and the aromatic monocyclic heterocyclic group may optionally be substituted by from 1 to 3 or so optionally halogenated $C_{1-4}$ alkyl groups e.g., methyl, chloromethyl, difluoromethyl, trichloromethyl, trifluoromethyl, ethyl and isopropyl groups, etc.), etc.

As preferred substituents for the monocyclic ring as ring C (e.g., benzene ring, etc.), for example, mentioned are a halogen atom (e.g., fluorine, chlorine and bromine atoms, etc.), an optionally halogenated $C_{1-6}$ alkyl group (e.g., methyl, chloromethyl, difluoromethyl, trichloromethyl, trifluoromethyl, ethyl, 2-bromoethyl, 2,2,2-trifluoroethyl, perfluoroethyl, propyl, isopropyl, 3,3,3-trifluoropropyl, butyl, sec-butyl, tert-butyl and perfluorobutyl groups, etc.), a nitro group, a hydroxyl group, an optionally halogenated $C_{1-6}$ alkoxy group (e.g., methoxy, difluoromethoxy, trifluoromethoxy, ethoxy, 2,2,2-trifluoroethoxy, perfluoroethoxy, propoxy, isopropoxy, 3,3,3-trifluoropropoxy and butoxy groups, etc.), an amino group, a mono- or di-$C_{1-4}$ alkylamino-substituted $C_{1-4}$ alkyl group (e.g., methylaminomethyl, dimethylaminomethyl, 2-methylaminoethyl and 2-dimethylaminoethyl groups, etc.), a mono- or di-$C_{1-4}$ alkylamino group (e.g., methylamino, ethylamino, dimethylamino and diethylamino groups, etc.), a $C_{1-4}$ alkoxy-carbonyl group (e.g., methoxycarbonyl and ethoxycarbonyl groups, etc.), a carboxyl group, a carbamoyl group, etc.

More preferred are a halogen atom (e.g., fluorine, chlorine and bromine atoms, etc.), an optionally halogenated $C_{1-4}$ alkyl group (e.g., methyl, chloromethyl, difluoromethyl, trichloromethyl, trifluoromethyl, ethyl, 2-bromoethyl, 2,2,2-trichloroethyl, 2,2,2-trifluoroethyl, perfluoroethyl, propyl, isopropyl and tert-butyl groups, etc.), an optionally halogenated $C_{1-4}$ alkoxy group (e.g., methoxy, trifluoromethoxy, ethoxy, 2,2,2-trichloroethoxy, 2,2,2-trifluoroethoxy, perfluoroethoxy and propoxy groups, etc.), a di-$C_{1-4}$ alkylamino group (e.g., dimethylamino and diethylamino groups, etc.), a $C_{1-3}$ acyloxy group (e.g., acetoxy group, etc.), a hydroxyl group, etc. Preferably, the number of the substituents is, for example, from 1 to 3 or so.

Especially, preferred are a halogen atom (e.g., fluorine, chlorine and bromine atoms, etc.), an optionally halogenated $C_{1-4}$ alkyl group (e.g., methyl, chloromethyl, difluoromethyl, trichloromethyl, trifluoromethyl, ethyl, 2-bromoethyl, 2,2,2-trichloroethyl, 2,2,2-trifluoroethyl, perfluoroethyl, propyl, isopropyl and tert-butyl groups, etc.), an optionally halogenated $C_{1-4}$ alkoxy group (e.g., methoxy, trifluoromethoxy, ethoxy, 2,2,2-trichloroethoxy, 2,2,2-trifluoroethoxy, perfluoroethoxy and propoxy groups, etc.) and the like.

The "heterocyclic ring" of the "heterocyclic ring optionally having substituents" includes, for example, from 5-membered to 10-membered heterocyclic rings having 1 to 4 hetero atoms of the same type or two different types, such as nitrogen, oxygen, sulfur atoms, etc., in addition to carbon atoms, etc. Concretely, the heterocyclic ring includes, for example;

(1) 5-membered or 6-membered, aromatic monocyclic heterocyclic rings, such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, etc.;
(2) 9-membered or 10-membered, aromatic, condensed heterocyclic rings, such as benzofuranyl, isobenzofuranyl, benzo[b]thienyl, indolyl, isoindolyl; 1H-indazolyl, benzimidazolyl, benzoxazolyl, 1,2-benzoisoxazolyl, benzothiazolyl, 1,2-benzoisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathinyl, thianthrenyl, phenanthridinyl, phenanthrolinyl, indolizinyl, pyrrolo[1,2-b]pyridazinyl, pyrazolo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-bipyridazinyl, etc.; and
(3) 5-membered to 10-membered, non-aromatic heterocyclic rings and the like, such as oxiranyl, azetidinyl, oxetanyl, thietanyl, pyrrolidinyl,tetrahydrofuryl, piperidyl, tetrahydropyranyl, morpholinyl, thiomorpholinyl, pyrazinyl, etc.

Of the above-mentioned heterocyclic rings (1) to (3), for example, 5-membered or 6-membered heterocyclic rings having from 1 to 3 hetero atoms, such as nitrogen, oxygen and sulfur atoms, etc., in addition to carbon atoms, are widely utilized. Such heterocyclic rings include, for example, furyl, thienyl, pyrrolyl, oxazolyl, isoxazolyl, imidazolyl, pyrazolyl, pyridyl, pyridazinyl, quinolyl, isoquinolyl, thiazolyl, thiadiazolyl, thiophenyl, etc.

As the substituents for the heterocyclic rings optionally having substituents, mentioned are substituents such as those as referred to above-mentioned "homocyclic rings optionally having substituents".

More preferably, ring C includes benzene rings optionally having substituents (especially benzene rings substituted by substituent), for example, benzene rings optionally substituted by 1 to 3 substituents selected from a halogen atom, an optionally halogenated $C_{1-4}$ alkyl group, an optionally halogenated $C_{1-4}$ alkoxy group, a di-$C_{1-4}$ alkylamino group, a $C_{1-3}$ acyloxy group and a hydroxyl group (especially, benzene rings substituted by such substituent(s)). Concretely, the preferred ring C includes, for example, optionally substituted benzene rings of the formula (C-1):

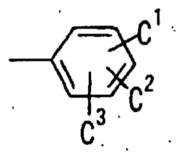
(C-1)

wherein $C^1$, $C^2$ and $C^3$ are the same or different and each represent a hydrogen atom, a halogen atom, an optionally halogenated $C_{1-4}$ alkyl group, an optionally halogenated $C_{1-4}$ alkoxy group, a mono- or di-$C_{1-4}$ alkylamino group, a $C_{1-3}$ acyloxy group or a hydroxyl group; and
optionally substituted benzene rings of the formula (C-2):

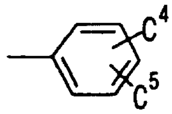

(C-2)

wherein $C^4$ and $C^5$ are the same or different and each represent a hydrogen atom, a halogen atom, an optionally halogenated $C_{1-4}$ alkyl group or an optionally halogenated $C_{1-4}$ alkoxy group.

The halogen atom, the optionally halogenated $C_{1-4}$ alkyl group, the optionally halogenated $C_{1-4}$ alkoxy group and the mono- or di-$C_{1-4}$ alkylamino group to be represented by any of $C^1$, $C^2$, $C^3$, $C^4$ and $C^5$ may be the same as the above-mentioned halogen atom, optionally halogenated $C_{1-4}$ alkyl group, optionally halogenated $C_{1-4}$ alkoxy group and mono- or di-$C_{1-4}$ alkylamino group, respectively.

Even more preferably, ring C includes, for example, benzene rings of the above-mentioned formulas (C-1) and (C-2) where $C^1$ to $C^5$ are substituents mentioned below:

(1) $C^1$, $C^2$ and $C^3$ are the same or different and each represent a halogen atom, an optionally halogenated $C_{1-4}$ alkyl group or an optionally halogenated $C_{1-4}$ alkoxy group ;
(2) $C^1$, $C^2$ and $C^3$ are the same or different and each represent a halogen atom or an optionally halogenated $C_{1-4}$ alkyl group;
(3) $C^1$, $C^2$ and $C^3$ are the same or different and each represent a halogen atom;
(4) $C^1$, $C^2$ and $C^3$ are the same or different and each represent an optionally halogenated $C_{1-4}$ alkyl group;
(5) $C^1$, $C^2$ and $C^3$ are the same or different and each represent an optionally halogenated $C_{1-4}$ alkoxy group;
(6) $C^4$ and $C^5$ are the same or different and each represent a halogen atom;
(7) $C^4$ and $C^5$ are the same or different and each represent an optionally halogenated $C_{1-4}$ alkyl group; or
(8) $C^4$ and $C^5$ are the same or different and each represent an optionally halogenated $C_{1-4}$alkoxy group.

As examples of the "optionally halogenated $C_{1-4}$ alkyl group", the "optionally halogenated $C_{1-4}$ alkoxy group" and the "halogen atom" in the above-mentioned embodiments (1) to (8), referred to are the same groups or atoms as those mentioned hereinabove.

Furthermore preferably, ring C includes, for example, benzene rings of the above-mentioned formula (C-2) wherein $C^4$ and $C^5$ are substituents mentioned below:

(a) one of $C^4$ and $C^5$ is a hydrogen atom and the other is a methoxy group;
(b) $C^4$ and $C^5$ are both chlorine atoms;
(c) one of $C^4$ and $C^5$ is a methoxy group and the other is an isopropyl group;
(d) one of $C^4$ and $C^5$ is a methoxy group and the other is a 1-methoxy-1-methylethyl group; or
(e) $C^4$ and $C^5$ are both trifluoromethyl groups.

**"ring Z":**

In the above-mentioned formulas, ring Z represents an optionally substituted heterocyclic ring containing nitrogen. Various substituents are referred to as substituents for ring Z, which include, for example, an alkyl group (e. g., a linear or branched alkyl group having from 1 to 6 carbon atoms, preferably a linear or branched alkyl group having from 1 to 4 carbon atoms, such as methyl, ethyl, propyl, isopropyl, butyl, isobutyl, sec-butyl and tert-butyl groups, etc .), an alkenyl group (e.g., a $C_{2-6}$ alkenyl group, preferably a $C_{2-4}$ alkenyl group, such as ethenyl, propenyl, isopropenyl, butenyl, isobutenyl sec-butenyl groups, etc.), an alkynyl group (e.g., a $C_{2-6}$ alkynyl group, preferably a $C_{2-4}$ alkynyl group, such. as ethynyl, propynyl, isopropynyl, butynyl, isobutynyl and sec-butynyl groups, etc.), acycloalkyl group (e. g., a $C_{3-8}$ cycloalkyl group, preferably a $C_{3-6}$ cycloalkyl group, such as cyclopropyl, cyclobutyl, cyclopentyl and cyclohexyl groups, etc.), a cycloalkyl-alkyl group (e.g., a $C_{3-6}$ cycloalkyl-$C_{1-4}$ alkyl group, such as cyclopropylmethyl, cyclopropylethyl and cyclohexylmethyl groups, etc.), an aryl group (e.g., a $C_{6-14}$ aryl group, preferably a $C_{6-10}$ aryl group, such as phenyl, 1-naphthyl, 2-naphthyl, anthryl and phenanthryl groups, etc., especially, phenyl group), a nitro group, a cyano group, a hydroxyl group, a $C_{1-4}$ alkoxy group (e.g., methoxy, ethoxy, propoxy, isopropoxy and butoxy groups, etc.), a $C_{1-4}$ alkylthio group (e.g., methylthio, ethylthio and propylthio groups, etc.), an amino group, a mono- or di-$C_{1-4}$ alkylamino group (e.g., methylamino, ethylamino, propylamino, dimethylamino and diethylamino groups, etc.), a cyclic amino group (e.g., a 5-membered to 9-membered cyclic amino group optionally having from 1 to 3 hetero atoms, such as oxygen and sulfur atoms, etc., in addition to nitrogen atom, concretely, for example, pyrrolidino, piperidino, morpholino and thiomorpholino groups, etc.), a $C_{1-4}$ alkyl-carbonylamino group (e.g., acetylamino, propionylamino and butyrylamino groups, etc.), a $C_{1-4}$ alkylsulfonylamino group (e.g., methylsulfonylamino and ethylsulfonylamino

groups, etc.), a $C_{1-4}$ alkoxy-carbonyl group (e.g., methoxycarbonyl, ethoxycarbonyl and propoxycarbonyl groups, etc.), a carboxyl group, a $C_{1-6}$ alkyl-carbonyl group (e.g., methylcarbonyl, ethylcarbonyl and propylcarbonyl groups, etc.); a carbamoyl group, a mono- or di-$C_{1-4}$ alkylcarbamoyl group (e.g., methylcarbamoyl and ethylcarbamoyl groups, etc.), a $C_{1-6}$ alkylsulfonyl group (e.g., methylsulfonyl, ethylsulfonyl and propylsulfonyl groups, etc.), an oxo group, a thioxo group, etc. The number of the substituents is, for example, from 1 to 5 or so, preferably 1, 2 or so, depending on the size of ring Z.

ring Z may be a heterocyclic ring optionally having at least one hetero atom selected from nitrogen, oxygen and sulfur atoms, in addition to Y and the nitrogen atom N, and is preferably an optionally oxoated ring.

ring Z includes a nitrogen containing heterocyclic ring represented by the formula (Z-1):

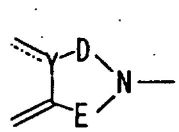

(Z-1)

wherein D and E represent groups from which ring Z as mentioned above is formed together with the nitrogen atom adjacent to E.

As D and E forming ring Z, at least one of them is an optionally oxoated alkylene group, an optionally oxoated oxyalkylene group, an optionally oxoated iminoalkylene group and the like. Preferable D and E are often an optionally oxoated alkylene group and an oxyalkylene group, respectively. The optionally oxoated alkylene group, oxyalkylene group and iminoalkylene group, which are represented by D and E, preferably have carbon atoms from which ring Z is formed to be a 5-membered to 12-membered ring, preferably a 5-membered to 9-membered ring. The number of the carbon atoms that constitute the alkylene groups of D and E may be the same or different.

Preferably, D includes, for example, optionally oxoated $C_{1-7}$ alkylene group, especially optionally oxoated $C_{1-5}$ alkylene group, $C_{1-7}$ oxyalkylene groups, especially $C_{1-5}$ oxyalkylene groups, $C_{1-7}$ iminoalkylene groups, especially $C_{1-5}$ imminoalkylene groups. More preferably, D includes an alkylene group of the formula $-(CH_2)_m-$ (wherein m is from 1 to 7), an oxyalkylene group of the formula $-O-(CH_2)_p-$ (wherein p is an integer of from 1 to 7), iminoalkylene group of the formula $-NH-(CH_2)_q-$ (wherein q is an integer of from 1 to 7). In these formulas, m and p are each preferably from 1 to 5, more preferably from 2 to 5.

Preferably, E includes, for example, optionally oxoated $C_{1-3}$ alkylene group, more preferably an optionally oxoated alkylene group having one or two carbon atoms, even more preferably an optionally oxoated methylene group.

The number of the oxo groups that are substitutable in the aforementioned ring z is not specifically limited but may be selected from 1 to 3 or so depending on the size of ring Z. Where ring Z is a 5-membered to 10-membered ring, the number of the substitutable oxo groups is 1, 2 or so. Oxo group(s) need only to substitute at least one of D and E. Preferably, oxo group(s) is/are substituted at E.

Preferably, in ring Z, D is an alkylene group or oxyalkylene group having from 1 to 5 carbon atoms, more preferably from 2 to. 5 carbon atoms, while E is an oxoated alkylene group having 1 or 2 carbon atoms, especially >C=O. Preferably, ring Z includes, for example, 5-membered to 9-membered nitrogen-containing heterocycles of the formula (Z-2):

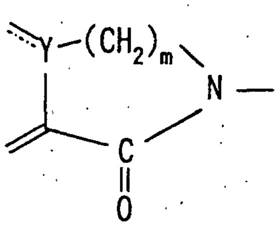

or

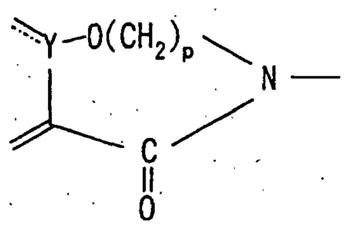

(Z-2)

wherein each m and p represents an integer of from 1 to 5.

"**n**":

In the above-mentioned formulas, n represents an integer of from 1 to 6, preferably an integer of from 1 to 3, especially preferably 1 or 2. More preferably, n is 1.

**Compounds (I) and (Ia):**

In compounds represented by the above-mentioned formulas (I) and (Ia), the combination of "ring M",

$$" -X \overline{\text{------}} Y < ",$$

"$R^a$", "$R^b$" , "ring A", "ring B", "ring C", "ring Z" and "n" is not specifically limited. These may be combined suitably to construct the compounds (I) and (Ia). Preferred compounds (I) and (Ia) are constructed by combining the above-mentioned preferred embodiments of "ring M",

$$" -X \overline{\text{------}} Y < ",$$

"$R^a$", "$R^b$","ring A", "ring B", "ring C", "ring Z" and "n".

Of the compounds of the above-mentioned formula (I), especially those of the formula (Ia), preferred are the following compounds (hereinafter to be also referred to as compound (1)) and pharmaceutically acceptable salts thereof.

Compounds wherein;

one of ring A and ring B is a 5-membered or 6-membered heterocyclic ring having one or two hetero atoms selected from nitrogen and sulfur atoms, in addition to carbon atoms, while the other is a benzene ring, and the rings A and B may have one or two substituents selected from a halogen atom and an optionally halogenated $C_{1-4}$ alkyl group;

ring C is a benzene ring optionally having from,1 to 3 substituents selected from a halogen atom, an optionally halogenated $C_{1-6}$ alkyl group (preferably, $C_{1-4}$ alkyl group) and an optionally halogenated $C_{1-6}$ alkoxy group (preferably, $C_{1-4}$ alkoxy group);

D that constitutes ring Z is $-(CH_2)_m-$(wherein m is an integer of from 1 to 7). or $-O-(CH_2)_p-$ (wherein p is an integer of from 1 to 7);

E that constitutes ring Z is >C=O;

$$-X \overline{\text{------}} Y<$$

is -CO-N< or -N=C<;

n is 1,

or pharmaceutically acceptable salts thereof.

The above-mentioned "5-membered or 6-membered heterocyclic ring" includes, for example, pyridine, pyrazine, pyrrole, thiophene, thiazole, tetrahydropyrazine, piperidine, etc. Concretely, ring A includes heterocyclic rings of the above-mentioned formula (A-5), etc., and ring B includes benzene rings of the above-mentioned formulas (B-7) and (B-8), especially the above-mentioned formula (B-10), etc.

The above-mentioned "halogen atom" includes, for example, fluorine, chlorine and bromine atoms, etc.; the "optionally halogenated $C_{1-4}$ alkyl group" includes, for example, methyl, chloromethyl, difluoromethyl, trichloromethyl, trifluoromethyl, ethyl, 2-bromoethyl, 2,2,2-trifluoroethyl, perfluoroethyl, propyl, 3,3,3-trifluoropropyl, isopropyl, 2-trifluoromethylethyl, butyl, 4,4,4-trifluorobutyl, isobutyl, sec-butyl and tert-butyl groups, etc.; the "optionally halogenated $C_{1-6}$ alkyl group" includes pentyl and hexyl groups, etc., in addition to the above-mentioned alkyl groups and halogenated alkyl groups.

The "optionally halogenated $C_{1-4}$ alkoxy group" includes, for example, methoxy, difluoromethoxy, trifluoromethoxy, ethoxy, 2,2,2-trifluoroethoxy, perfluoroethoxy, propoxy, isopropoxy, butoxy, 4,4,4-trifluorobutoxy, isobutoxy, sec-butoxy and tert-butoxy groups, etc.; and the "optionally halogenated $C_{1-6}$ alkoxy group" includes pentyloxy and hexyloxy groups, etc., in addition to the above-mentioned alkoxy groups and halogenated alkoxy groups.

Of the compounds of the above-mentioned formula (I), especially those of the formula:(Ia), also preferred are the following compounds and pharmaceutically acceptable salts thereof.

Compounds wherein; ring A is a 5-membered or 6-membered heterocyclic ring having one nitrogen atom or one sulfur atom, in addition to carbon atoms, for example, a heterocyclic ring of the formula (A-7):

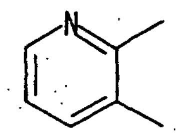, 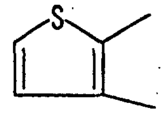 (A-7)

ring B is a benzene ring optionally having 1 to 3 substituents selected from a halogen atom and an optionally halogenated $C_{1-4}$ alkyl group;
ring C is a benzene ring optionally having 1 to 3 substituents selected from a halogen atom, an optionally halogenated $C_{1-4}$ alkyl group and an optionally halogenated $C_{1-4}$ alkoxy group;
D that constitutes ring Z is $-(CH_2)_m-$ (wherein m is an integer of from 1 to 7) or $-O-(CH_2)_p-$ (wherein p is integer of from 1 to 7);
E that constitutes ring Z is >C=O;

$$— X \overline{\text{------}} Y <$$

is -CO-N<;
n is 1,
or pharmaceutically acceptable salts thereof.

AS examples of the "halogen atom", the "optionally halogenated $C_{1-4}$ alkyl group" and the "optionally halogenated $C_{1-4}$ alkoxy group", the atoms and groups similar to those mentioned above for compound (1) can be mentioned.

More preferably, compounds wherein; $R^a$ and $R^b$ are the same or different and each represent a hydrogen atom or a $C_{1-6}$ alkyl group optionally substituted by (1) $C_{1-6}$ alkoxy group, (2) $C_{1-6}$ alkylthio group, (3) amino group, (4) $C_{1-7}$ acylamino group, (5) mono- or di-$C_{1-6}$ alkylamino group, (6) $C_{3-10}$ cyclic amino group, (7) 5-membered or 6-membered cyclic amino group optionally substituted by $C_{1-6}$ alkyl group, (8) $C_{1-6}$ alkylsulfonylamino group or (9) $C_{1-6}$ alkylcarbonyloxy group; or
$R^a$ and $R^b$ are bonded to each other to form pyridine ring which is optionally substituted by 1 to 3 substituents selected from a halogen atom and a $C_{1-4}$ alkyl group;
ring B is a benzene ring optionally having 1 to 3 substituents selected from (1) a halogen atom, (2) an optionally halogenated $C_{1-4}$ alkyl group and (3) an optionally halogenated $C_{1-4}$ alkoxy group;
ring C is a benzene ring optionally having 1 to 3 substituents selected from (1) a halogen atom, (2) an optionally halogenated $C_{1-4}$ alkyl group, (3) an optionally halogenated $C_{1-4}$ alkoxy group, (4) an amino group optionally substituted by $C_{1-4}$ alkyl group, (5) a $C_{1-3}$ acyloxy group and (6) a hydroxyl group;
ring Z is a 5-membered to 10-membered nitrogen-containing optionally oxoated heterocyclic ring and optionally substituted $C_{1-4}$ alkyl group or a hydroxyl group;

$$— X \overline{\text{------}} Y <$$

is -CO-N< or -N=C<;
and n is 1, or salts thereof.

Preferred compounds (T) include, for example, the compounds represented by the following formula:

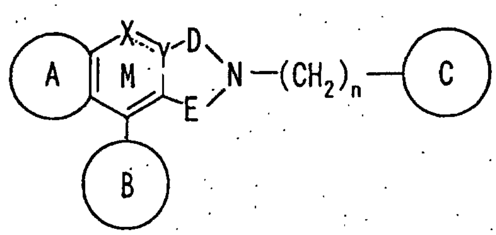

wherein D and E represent optionally oxoated alkylene groups and the other symbols are as defined above, salts thereof and prodrugs thereof.

Preferably, D and E each represent a $C_{1-3}$ alkylene group optionally substituted by one oxo group.

More preferred compounds (T) include, for example, the compounds represented by the following formula:

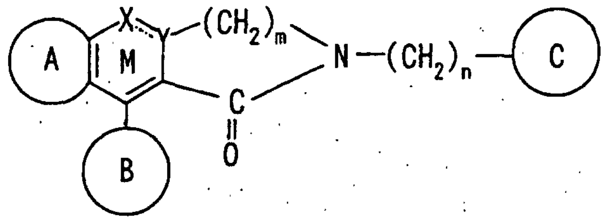

wherein m represents an integer of from 1 to 7, and the other symbols are as defined above, salts thereof .and prodrugs thereof.

m is preferably an integer of from 2 to 5.

In the above-mentioned formulas, preferably $R^a$ and $R^b$ each represent a hydrogen atom or a substituent selected from the group consisting of

(I) a halogen atom,
(2) a $C_{1-6}$ alkyl group optionally having from 1 to 5 substituents selected from the group consisting of

(a) a hydroxyl group,
(b) a $C_{1-6}$ alkoxy group,
(c) a $C_{1-6}$ alkylthio group,
(d) an amino group,
(e) a $C_{1-7}$ acylamino group,
(f) a mono- or di-$C_{1-6}$ alkylamino group,
(g) a mono- or di-$C_{3-8}$ cycloalkylamino group,
(h) a 5-membered to 9-membered cyclic amino group which may have 1 to 3, one or two kinds of hetero atoms selected from the group consisting of oxygen and sulfur atoms in addition to nitrogen atom and which may be substituted by $C_{1-6}$ alkyl group,
(i) a $C_{1-4}$ alkylsulfonylamino group,
(j) a $C_{1-6}$ alkyl-carbonyloxy group and
(k) a halogen atom,

(3) a 5-membered to 9-membered (preferably 6-membered) cyclic amino group which may have 1 to 3 hetero atoms (preferably 1 or 2) selected from the group consisting of oxygen and sulfur atoms, in addition to nitrogen atom and which may be substituted by $C_{1-6}$ alkyl group,
(4) a carboxyl group,
(5) carbamoyl group,
(6) a mono- or di-$C_{1-6}$ alkylcarbamoyl group; or

$R^a$ and $R^b$ are bonded to each other to form ring A, and the ring A is a 5-membered to 9-membered aromatic heterocyclic ring having from 1 to 3, one or two kinds of hetero atoms selected from the group consisting of nitrogen, sulfur and oxygen atoms, in addition to carbon atoms (preferably pyridine ring), which may be substituted by $C_{1-6}$ alkyl group; the ring B is a $C_{6-14}$ aryl group (preferably benzene ring) which may be substituted by substituents selected from the group consisting of (i) a $C_{1-6}$ alkyl group optionally substituted by a hydroxyl group, (ii) a $C_{1-6}$ alkylcarbonyl group (including formyl) and (iii) a carboxyl group;
the ring C is a $C_{6-14}$ aryl group (preferably benzene ring) which may be substituted by 1 to 3 substituents selected from the group consisting of (i) a halogen atom, (ii) optionally halogenated $C_{1-10}$ alkyl group and (iii) $C_{1-10}$ alkoxy group;
the ring Z is a 5-membered to 12-membered heterocyclic ring optionally having at least one hetero atom selected from the group consisting of nitrogen, oxygen and sulfur atoms, in addition to Y and nitrogen atom, which may be substituted by 1 to 3 substituents selected from the group consisting of (i) a $C_{1-6}$ alkyl group, (ii) a hydroxyl group and (iii) oxo group.

The compounds represented by the above-mentioned formula (I) may form a salt, and when they are used as pharmaceutical products, the salt is preferably a pharmaceutically acceptable salt.

Examples of the pharmaceutically acceptable salt include salts with inorganic acids, such as hydrochloric acid, sulfuric acid, phosphoric acid, diphosphoric acid, hydrobromic acid, nitric acid, etc., or salts with organic acids, such as acetic acid, malic acid, maleic acid, fumaric acid, tartaric acid, succinic acid, citric acid, lactic acid, methanesulfonic acid, p-toluenesulfonic acid, palmitic acid, salicylic acid, stearic acid, etc.

The compound (T) to be used in the present invention include stereoisomers such as cis- and trans-isomers, etc., racemates, as well as optically active forms such as R-forms, S-forms, etc. Depending on the size of ring z, conformation-dependent isomers may be generated, which are also encompassed in compound (T).

Preferable compounds included incompounds (I) and (Ia) are shown in the following.

(1) 7-[3,5-bis(trifluoromethyl)benzyl]-6,7,8,9-tetrahydro-5-(4-methylphenyl)-6,11-dioxo-11H-pyrazino[2,1-g][1,7] naphthyridine (hereinafter sometimes abbreviated as compound No. 1)

(2) 7-[3,5-bis(trifluoromethyl)benzyl]-6,7,8,9,10,12-hexahydro-5-(4-methylphenyl)-6,12-dioxo-[1,4]diazepino[2,1-g][1,7]naphthyridine (hereinafter sometimes abbreviated as compound No. 2)

(3) 7-[3,5-bis(trifluoromethyl)benzyl]-6,7,8,9,10,11-hexahydro-5-(4-methylphenyl)-6,13-dioxo-13H-[1,4]diazocino [2,1-g][1,7]naphthyridine

(4) 6,7,8,9,10,12-hexahydro-7-(2-methoxybenzyl)-5-(4-methylphenyl)-6,12-dioxo-[1,4]diazepino[2,1-g][1,7]naphthyridine

(5) 6,7,8,9,10,11-hexahydro-7-(2-methoxybenzyl)-5-(4-methylphenyl)-6,13-dioxo-13H-[1,4]diazocino[2,1-g] [1,7] naphthyridine

(6) 7-[3,5-bis(trifluoromethyl)benzyl]-6,7,8,9,10,11,12,14-octahydro-5-(4-methylphenyl)-6,14-dioxo[1,4]diazonino [2,1-g] [1,7]naphthyridine

(7) 7-[3,5-bis(trifluoromethyl)benzyl]-6,7,8,9,10,12-hexahydro-6,12-dioxo-5-phenyl[1,4]diazepino[2,1-g] [1,7] naphthyridine

(8) 7-[3,5-bis(trifluoromethyl)benzyl]-6,7,8,9,10,11-hexahydro-6,13-dioxo-5-phenyl-13H-[1,4]diazocino[2,1-g][1,7] naphthyridine

(9) 7-[3,5-bis(trifluoromethyl)benzyl]-6,7,8,9,10,11 hexahydro-5-(4-methylphenyl)-6,13-dioxo-13H-[1,4]diazocino [1,2-b][2,7]naphthyridine

(10) 7-[3,5-bis(trifluoromethyl)benzyl]-1,2,3,4,6,7,8,9,10,11-decahydro-2-methyl-5-(4-methylphenyl)-6,13-dioxo-13H-[1,4]diazocino[1,2-b][2,7]naphthyridine

(11)4-[3,5-bis(trifluoromethyl)benzyl]-2,3,4,5-tetrahydro-5-oxo-6-phenylpyrido[3,2-f][1,4]oxazepine

(12) (9R)-7-[3,5-bis(trifluoromethyl)benzyl]-6,1,8,9,10,12-hexahydro-9-methyl-5-(4-methylphenyl)-6,12-dioxo[1,4] diazepino[2,1-g][1,7]naphthyridine

(13) (9S)-7-[3,5-bis(trifluoromethyl)benzyl]-6,7,8,9,10,12-hexahydro-9-methyl-6,12-dioxo-5-phenyl[1,4]diazepino [2,1-g][1,7]naphthyridine

(14) (9S)-7-[3,5-bis(trifluoromethyl)benzyl]-6,7,8,9,10,12-hexahydro-9-methyl-5-(4-methylphenyl)-6,12-dioxo[1,4] diazepino[2,1-g][1,7]naphthyridine

(15) (±)-7-[3,5-bis(trifluoromethyl)benzyl]-6,7,8,9,10,11-hexahydro-9-methyl-6,13-dioxo-5-phenyl-13H-[1,4]diazocino[2,1-g][1,7]naphthyridine

(16) (±)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine

(17) (9R)-7-[3,5-bis(trifluoromethyl)benzyl]-6,7,8,9,10,11-hexahydro-9-methyl-6,13-dioxo-5-phenyl-13H-[1,4]diazocino[2,1-g][1,7]naphthyridine

(18) (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine (hereinafter sometimes abbreviated as compound No. 3)

(19) (9S)-7-[3,5-bis(trifluoromethyl)benzyl]-6,7,8,9,10,11-hexahydro-9-methyl-6,13-dioxo-5-phenyl-13H-[1,4]diazocino[2,1-g] [1,7]naphthyridine

(20) (9S)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g] [1,7]naphthyridine

(21) 4-[3,5-bis(trifluoromethyl)benzyl]-2,3,4,5-tetrahydro-5-oxo-6-phenyl-1H-pyrido[2,3-e] [1,4]diazepine

(22) 5-[3,5-bis(trifluoromethyl)benzyl]-2,3,4,5-tetrahydro-6-oxo-7-phenyl-6H-pyrido[2,3-b] [1,5]oxazocine

(23) 4-[3,5-bis(trifluoromethyl)benzyl]-2,3,4,5-tetrahydro-7-methyl-5-oxo-6-phenylpyrido[3,2-f][1,4]oxazepine

(24) 5-[3,5-bis(trifluoromethyl)benzyl]-2,3,4,5-tetrahydro-8-methyl-6-oxo-7-phenyl-6H-pyrido[2,3-b][1,5]oxazocine

(25) (±)-7-[3,5-bis(trifluoromethyl)benzyl]-6,7,8,9,10,12-hexahydro-9-hydroxy-5-(4-methylphenyl)-6,12-dioxo[1,4] diazepino[2,1-g][1,7]naphthyridine

(26) 7-benzyl-6,7,8,9,10,12-hexahydro-6,12-dioxo-5-phenyl[1,4]diazepino[2,1-g][1,7]naphthyridine

(27) 7-benzyl-6,7,8,9,10,11-hexahydro-6,13-dioxo-5-phenyl-13H-[1,4]diazocino[2,1-g] [1,7]naphthyridine

(28) 7-benzyl-6,7,8,9,10,11,12,14-octahydro-6,14-dioxo-5-phenyl[1,4]diazonino[2,1-g] [1,7]naphthyridine

(29) 7-(3,4-dichlorobenzyl)-6,7,8,9,10,12-hexahydro-6,12-dioxo-5-phenyl[1,4]diazepino[2,1-g][1,7]naphthyridine

(30) 7-(3,4-dichlorobenzyl)-6,7,8,9,10,11-hexahydro-6,13-dioxo-5-phenyl-13H-[1,4]diazocino[2,1-g][1,7]naphthyridine

(31) (S)-5-[3,5-bis(trifluoromethyl)benzyl]-2,3,4,5-tetrahydro-3,8-dimethyl-6-oxo-7-phenyl-6H-pyrido[2,3-b][1,5]oxazocine
(32) (R)-5-[3,5-bis(trifluoromethyl)benzyl]-2,3,4,5- . tetrahydro-3,8-dimethyl-6-oxo-7-phenyl-6H-pyrido[2,3-b][1,5] oxazocine
(33) 4-[3,5-bis(trifluoromethyl)benzyl]-2,3,4,5-tetrahydro-8-methyl-5-oxo=6-phenylpyrido[3,2-f] [1,4]oxazepine
(34) 5-[3,5-bis(trifluoromethyl)benzyl]-2,3,4,5-tetrahydro-9-methyl-6-oxo-7-phenyl-6H-pyrido[2,3-b] [1,5]oxazocine
(35) 4-[3,5-bis(trifluoromethyl)benzyl]-2,3,4,5-tetrahydro-8-methyl-5-oxo-6-phenylpyrido[3,2-f] [1,4]oxazepine 9-oxide
(36) 5-[3,5-bis(trifluoromethyl)benzyl]-2,3,4,5-tetrahydro-9-methyl-6-oxo-7-phenyl-6H-pyrido[2,3-b] [1,5]oxazocine 10-oxide
(37) 8-acetoxymethyl-4-[3,5-bis(trifluoromethyl)benzyl]-2,3,4,5-tetrahydro-5-oxo-6-phenylpyrido-[3,2-f] [1,4]oxazepine
(38) 9-acetoxymethyl-5-[3,5-bis(trifluoromethyl)benzyl] 2,3,4,5-tetrahydro-6-oxo-7-phenyl-6H-pyrido[2,3-b][1,5] oxazocine
(39) 4-[3,5-bis(trifluoromethyl)benzyl]-8-chloromethyl-2,3,4,5-tetrahydro-5-oxo-6-phenylpyrido[3,2-f][1,4]oxazepine
(40) 4-[3,5-bis(trifluoromethyl)benzyl]-2,3,4,5-tetrahydro-8-methoxymethyl-5-oxo-6-phenylpyrido[3,2-f][1,4]oxazepine
(41) 4-[3,5-bis(trifluoromethyl)benzyl]-2,3,4,5-tetrahydro-8-(2-methylethyl)-5-oxo-6-phenylpyrido[3,2-f][1,4]oxazepine
(42) 4-[3,5-bis(trifluoromethyl)benzyl]-2,3,4,5-tetrahydro-8-methylthiomethyl-5-oxo-6-phenylpyrido[3,2-f] [1,4]oxazepine
(43) 8-aminomethyl-4-[3,5-bis(trifluoromethyl)benzyl]-2,3,4,5-tetrahydro-5-oxo-6-phenylpyrido[3,2-f] [1,4]oxazepine
(44) 4-[3,5-bis(trifluoromethyl)benzyl]-2,3,4,5-tetrahydro-8-methylaminomethyl-5-oxo-6-phenylpyrido[3,2-f][1,4] oxazepine
(45) 4-[3,5-bis(trifluoromethyl)benzyl]-8-dimethylaminomethyl-2,3,4,5-tetrahydro-5-oxo-6-phenylpyrido[3,2-f][1,4] oxazepine
(46) 4-[3,5-bis(trifluoromethyl)benzyl]-8-cyclopropylaminomethyl-2,3,4,5-tetrahydro-5-oxo-6-phenylpyrido[3,2-f] [1,4]oxazepine
(47) 4-[3,5-bis(trifluoromethyl)benzyl]-2,3,4,5-tetrahydro-8-(N-methylpiperazinomethyl)-5-oxo-6-phenylpyrido[3,2-f][1,4]oxazepine
(48) 8-acetylaminomethyl-4-[3,5-bis(trifluoromethyl)benzyl]-2,3,4,5-tetrahydro-5-oxo-6-phenylpyrido[3,2-f][1,4] oxazepine
(49) 4-[3,5-bis(trifluoromethyl)benzyl]-2,3,4,5-tetrahydro-8-methanesulfonylaminomethyl-5-oxo-6-phenylpyrido[3,2-f][1,4]oxazepine
(50) 6-[3,5-bis(trifluoromethyl)benzyl]-5,6,7,8-hexahydro-3,9-dimethyl-5,10-dioxo-4-phenylpyrido[2,3-f][1,4]diazocine
(51) 6-[3,5-bis(trifluoromethyl)benzyl]-5,6,7,8,9,10-hexahydro-9-methyl-5,10-dioxo-4-phenylpyrido[2,3-f][1,4]diazocine
(52) 6-benzyl-5,6,7,8,9,10-hexahydro-3,9-dimethyl-5,10-dioxo-4-phenylpyrido[2,3-f][1,4]diazocine
(53) 6-[3,5-bis(trifluoromethyl)benzyl]-9-ethyl-5,6,7,8,9,10-hexahydro-3-methyl-5,10-dioxo-4-phenylpyrido[2,3-f] [1,4]diazocine
(54) 6-[3,5-bis(trifluoromethyl)benzyl]-6,7,8,9,10,11-hexahydro-3,10-dimethyl-5,11-dioxo-4-phenyl-5H-pyrido[2,3-g][1,5]diazonine
(55) 4-[3,5-bis(trifiuoromethyl)benzyl]-8-hydroxymet,hyl-2,3,4,5-tetrahydro-5-oxo-6-phenylpyrido[3,2-f][1,4]oxazepine
(56) 4-[3,5-bis(trifluoromethyl)benzyl]-2,3,4,5-tetrahydro-5-oxo-6-phenylpyrido[3,2-f][1,4)oxazepine-8-carboxylic acid
(57) 4-[3,5-bis(trifluoromethyl)benzyl]-2,3,4,5-tetrahydro-5-oxo-6-phenylpyrido[3,2-f] [1,4]oxazepine-8-carboxamide
(58) 4-[3,5-bis(trifluoromethyl)benzyl]-N-methyl-2,3,4,5-tetrahydro-5-oxo-6-phenylpyrido[3,2-f] [1,4]oxazepine-8-carboxamide
(59) 4-[3,5-bis(trifluoromethyl)benzyl]-N,N-dimethyl-2,3,4,5-tetrahydro-5-oxo-6-phenylpyrido[3,2-f][1,4]oxazepine-8-carboxamide
(60) 4-[3,5-bis(trifluoromethyl)benzyl]-N-n-butyl-2,3,4,5-tetrahydro-5-oxo-6-phenylpyrido[3,2-f][1,4]oxazepine-8-carboxamide
(61) A-[3,5-bis(trifluoromethyl)benzyl]-2,3,4,5-tetrahydro-5-oxo-6-phenyl-8-piperidinocarbonylpyrido[3,2-f][1,4]oxazepine

(62) 4-[3,5-bis(trifluoromethyl)benzyl]-2,3,4,5-tetrahydro-8-morpholinocarbonyl-5-oxo-6-phenylpyrido[3,2-f][1,4] oxazepine

(63) 4-[3,5-bis(trifluoromethyl)benzyl]-2,3,4,5-tetrahydro-8-[1-(4-methylpiperazinyl)carbonyl]-5-oxo-6-phenylpyrido[3,2-f][1,4]oxazepine

(64) 2,3,4,5-tetrahydro-5-oxo-6-phenyl-4-(3,4,5-trimethoxybenzyl)pyrido[3,2-f][1,4]oxazepine

(65) 4-(3,4-dichlorobenzyl)-2,3,4,5-tetrahydro-5-oxo-6-phenylpyrido[3,2-f][1,4]oxazepine

(66) 4-(3,4-dimethoxybenzyl)-2,3,4,5-tetrahydro-5-oxo-6-phenylpyrido[3,2-f][1,4]oxazepine

(67) 4-benzyl-2,3,4,5-tetrahydro-5-oxo-6-phenylpyrido[3,2-f][1,4]oxazepine

(68) 2,3,4,5-tetrahydro-6-oxo-7-phenyl-5-(3,4,5-trimethoxybenzyl)-6H-pyrido[2,3-b][1,5]oxazocine

(69) (S)-5-benzyl-2,3,4,5-tetrahydro-3-methyl-6-oxo-7-phenyl-6H-pyrido[2,3-b][1,5]oxazocine

(70) (R)-5-benzyl-2,3,4,5-tetrahydro-3-methyl-6-oxo-7-phenyl-6H-pyrido[2,3-b][1,5]oxazocine

(71) (S)-5-[3,5-bis(trifluoromethyl)benzyl]-2,3,4,5-tetrahydro-3-methyl-6-oxo-7-phenyl-6H-pyrido[2,3-b][1,5]oxazocine.

(72) (R)-5-[3,5-bis(trifluoromethyl)benzyl]-2,3,4,5-tetrahydro-3-methyl-6-oxo-7-phenyl-6H-pyrido[2,3-b][1,5]oxazocine

(73) 7-benzyl-6,7,8,9,10,11-hexahydro-5-(4-methylphenyl)-6,13-dioxo-13H-[1,4]diazocino[2,1-g][1,7]naphthyridine

(74) (9R)-7-benzyl-6,7,8,9,10,11-hexahydro-9-methyl-5-(4-methylphenyl)-6,13-dioxo-13H-[1,4]diazocino[2,1-g][1,7]naphthyridine

(75) (9S)-7-benzyl-6,7,8,9,10,11-hexahydro-9-methyl-5-(4-methylphenyl)-6,13-dioxo-13H-[1,4]diazocino[2,1-g) [1,7]naphthyridine

(76) (9R)-6,7,8,9,10,11-hexahydro-9-methyl-5-(4-methylphenyl)-6,13-dioxo-7-(3,4,5-trimethoxybenzyl)-13H-[1,4]diazocino[2,1-g] [1,7]naphthyridine

(77) (9S)-6,7,8,9,10,11-hexahydro-9-methyl-5-(4-methylphenyl)-6,13-dioxo-7-(3,4,5-trimethoxybenzyl)-13H-[1,4]diazocino[2,1-g][1,7]naphthyridine

(78) (9R)-7-(3,5-dimethoxybenzyl)-6,7,8,9,10,11-hexahydro-9-methyl-5-(4-methylphenyl)-6,13-dioxo-13H-[1,4]diazocino[2,1-g][1,7]naphthyridine

(79) 4-benzyl-2,3,4,5-tetrahydro-5-oxo-6-(4-methylphenyl)pyrido[3,2-f][1,4]oxazepine

(80) 4-[3,5-bis(trifluoromethyl)benzyl]-2,3,4,5-tetrahydro-5-oxo-6-(4-methylphenyl)pyrido[3,2-f][1,4]oxazepine

(81) (S)-5-benzyl-2,3,4,5-tetrahydro-3-methyl-7-(4-methylphenyl)-6-oxo-6H-pyrido[2,3-b][1,5]oxazocine

(82) (S)-5-[3,5-bis(trifluoromethyl)benzyl-2,3,4,5-tetrahydro-3-methyl-7-(4-methylphenyl)-6-oxo-6H-pyrido[2,3-b] [1,5]oxazocine

(83) (9R)-7-[3,5-bis(trifluoromethyl)benzyl]-6,7,8,9,10,11-hexahydro-5-(4-hydroxymethylphenyl)-9-methyl-6,13-dioxo-13H-[1,4]diazocino[2,1-g][1,7]naphthyridine

(84) (9R)-7-[3,5-bis(trifluoromethyl)benzyl]-5-(4-formylphenyl)-6,7,8,9,10,11-hexahydro-9-methyl-6,13-dioxo-13H-[1,4]diazocino[2,1-g][1,7]naphthyridine

(85) (9R)-7-[3,5-bis(trifluoromethyl)benzyl]-5-(4-formylphenyl)-6,7,8,9,10,11-hexahydro-9-methyl-6,13-dioxo-13H-[1,4]diazocino[2,1-g] [1,7]naphthyridine

(86) (9R)-7-[3,5-bis(trifluoromethyl)benzyl]-5-(4-carboxyphenyl)-6,7,8,9,10,11-hexahydro-9-methyl-6,13-dioxo-13H-[1,4]diazocino[2,1-g] [1,7]naphthyridine

(87) (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g] [1,7]naphthyridine N-oxide

(88) (9R)-7-[3,5-bis(trifluoromethyl)benzyl]-6,7,8,9,10,11-hexahydro-5-(4-hydroxymethylphenyl)-9-methyl-6,13-dioxo-13H-[1,4]diazocino[2,1-g] [1,7]naphthyridine N-oxide

(89) (9R)-7-[3,5-bis(trifluoromethyl)benzyl]-5-(4-carboxyphenyl)-6,7,8,9,10,11-hexahydro-9-methyl-6,13-dioxo-13H-[1,4]diazocino[2,1-g] [1,7]naphthyridine N-oxide

(90) (9S)-[10,10,11,11-$^2H_4$]-7-[3,5-bis(trifluoromethyl)benzyl]-8,9,10,11-tetrahydro-5-(4-hydroxymethylphenyl)-9-methyl-7H-[1,4]diazocino[2,1-g][1,7]naphthyridine-6,13-dione ($d_4$ form of the compound of the above-mentioned (83))

(91) (9S)-[10,10,11,11-$^2H_4$]-7-[3,5-bis(trifluoromethyl)benzyl]-5-(4-formylphenyl)-8,9,10,11-tetrahydro-9-methyl-7H-[1,4]diazocino[2,1-g][1,7]naphthyridine-6,13-dione ($d_4$ form of the compound of the above-mentioned (84))

(92)(9S)-[10,10,11,11-$^2H_4$]-7-[3,5-bis(trifluoromethyl)benzyl]-5-(4-carboxyphenyl)-8,9,10,11-tetrahydro-9-methyl-7H-[1,4]diazocino[2,1-g][1,7]naphthyridine-6,13-dione ($d_4$ form of the compound of the above-mentioned (86))

(93) (9R)-7-[3,5-di(benzyloxy)benzyl]-6,7,8,9,10,11-hexahydro-9-methyl-5-(4-methylphenyl)-6,13-dioxo-13H-[1,4]diazocino[2,1-g][1,7]naphthyridine

(94) (9R)-7-(3,5-dihydroxybenzyl)-6,7,8,9,10,11-hexahydro-9-methyl-5-(4-methylphenyl)-6,13-dioxo-13H-[1,4]diazocino[2,1-g][1,7]naphthyridine

(95) (9R)-7-(3,5-diethoxybenzyl)-6,7,8,9,10,11-hexahydro-9-methyl-5-(4-methylphenyl)-6,13-dioxo-13H-[1,4]diazocino[2,1-g][1,7]naphthyridine

(96) (9R)-7-[3,5-di(1-methylethyloxy)benzyl]-6,7,8,9,10,11-hexahydro-9-methyl-5-(4-methylphenyl)-6,13-dioxo-13H-[1,4]diazocino[2,1-g] [1,7]naphthyridine

(97) (9R)-7-(3,5-dimethoxybenzyl)-6,7,8,9,10,11-hexahydro-9-methyl-6,13-dioxo-5-phenyl-13H-[1,4]diazocino[2,1-g] [1,7]naphthyridine

(98) (9R)-7-[3,5-bis(trifluoromethyl)benzyl]-5-(4-chlorophenyl)-6,7,8,9,10,11-hexahydro-9-methyl-6,13-dioxo-13H-[1,4]diazocino[2,1-g][1,7]naphthyridine

(99) (9R)-7-[3,5-bis(trifluoromethyl)benzyl]-5-(3,4-dichlorophenyl)-6,7,8,9,10,11-hexahydro-9-methyl-6,13-dioxo-13H-[1,4]diazocino[2,1-g][1,7]naphthyridine

(100) (9R)-7-(3,5-dimethoxybenzyl)-5-(3,4-dichlorophenyl)-6,7,8,9,10,11-hexahydro-9-methyl-6,13-dioxo-13H-[1,4]diazocino[2,1-g] [1,7]naphthyridine

(101) (9R)-7-(3,5-dimethylbenzyl)-6,7,8,9,10,11-hexahydro-9-methyl-5-(4-methylphenyl)-6,13-dioxo-13H-[1,4]diazocino[2,1-g] [1,7]naphthyridine

(102) (9R)-7-(3,5-dichlorobenzyl)-6,7,8,9,10,11-hexahydro-9-methyl-5-(4-methylphenyl)-6,13-dioxo-13H-[1,4]diazocino[2,1-g][1,7]naphthyridine

(103) (9R)-5-(3,4-dichlorophenyl)-7-(3,5-dimethylbenzyl)-6,7,8,9,10,11-hexahydro-9-methyl-6,13-dioxo-13H-[1,4]diazocino[2,1-g][1,7]naphthyridine

(104) (9R)-7-(3,5-dimethoxybenzyl)-5-(4-fluorophenyl)-6,7,8,9,10,11-hexahydro-9-methyl-6,13-dioxo-13H-[1,4]diazocino[2,1-g] [1,7]naphthyridine

(105) (9R)-5-(4-chlorophenyl)-7-(3,5-dimethoxybenzyl)-6,7,8,9,10,11-hexahydro-9-methyl-6,13-dioxo-13H-[1,4]diazocino[2,1-g] [1,7]naphthyridine

(106) (9R)-7-[3,5-bis(trifluoromethyl)benzyl]-5-(4-fluorophenyl)-6,7,8,9,10,11-hexahydro-9-methyl-6,13-dioxo-13H-[1,4]diazocino[2,1-g] [1,7]naphthyridine

(107) (±)-7-[3,5-bis(trifluoromethyl)benzyl]-9-ethyl-6,7,8,9,10,11-hexahydro-5-(4-methylphenyl)-6,13-diox6-13H-[1,4]diazocino[2,1-g][1,7]naphthyridine

(108) (±)-7-[3,5-bis(trifluoromethyl)benzyl]-6,7,8,9,10,11-hexahydro-9-(1-methylethyl)-5-(4-methylphenyl)-6,13-dioxo-13H-[1,4]diazocino[2,1-g][1,7]naphthyridine

(109) (±)-5-(3,4-dichlorophenyl)-7-(3,5-dimethoxybenzyl)-9-ethyl-6,7,8,9,10,11-hexahydro-6,13-dioxo-13H-[1,4]diazocino[2,1-g][1,7]naphthyridine

(1,10) (±)-5-(3,4-dichlorophenyl)-7-(3,5-dimethoxybenzyl)-6,7,8,9,10,11-hexahydro-9-(1-methylethyl)-6,13-dioxo-13H-[1,4]diazocino[2,1-g][1,7]naphthyridine

**Production method of compound and salt thereof**

The compound (T) can be produced according to the methods described in JP-A-9-263585 and JP-A-10-109989.

The compounds of the above-mentioned (1)-(82) can be produced based on the description of Examples of JP-A-9-263585. The compounds of the above-mentioned (83)-(92) can be produced based on the description of Examples of JP-A-10-109989. The compounds of the above-mentioned (93)-(110) can be produced according to the description of Examples of JP-A-9-263585 or JP-A-10-109989.

Of the compounds (T), a prodrug means a compound which is converted to compound (I) under physiological conditions in living organisms as a result of a reaction with an enzyme, gastric acid and the like. In other words, it means a compound that undergoes enzymatic oxidation, reduction, hydrolysis and the like into compound (I) and a compound that undergoes hydrolysis and the like by gastric acid and the like into compound (I).

Examples of the prodrug of compound (I) include a compound wherein an amino group of compound (I) is acylated, alkylated or phosphorylated (e.g., compound wherein amino group of compound (I) is eicosanoylated, alanylated, pentylaminocarbonylated, (5-methyl-2-oxo-1,3-dioxolen-4-yl)methoxycarboriylated, tetrahydrofuranylated, pyrrolidylmethylated, pivaloyloxymethylated, tert-butylated, etc.); a compound wherein a hydroxyl group of compound (I) is acylated, alkylated, phosphorylated, borated (e.g., compound wherein hydroxyl group of compound (I) is acetylated, palmitoylated, propanoylated, pivaloylated, succinylated, fumarylated, alanylated, dimethylaminomethylcarbonylated, etc.); a compound wherein a carboxyl group of compound (I) is esterified, amidated (e.g., compound wherein a carboxyl groups of compound (I) is ethyl esterified, phenyl esterified, carboxymethyl, esterified, dimethylaminomethyl esterified, pivaloyloxymethyl esterified, ethoxycarbonyloxyethyl esterified, phthalidyl esterified, (5-methyl-2-oxo-1,3-dioxolen-4-yl)methyl esterified, cyclohexyloxycarbonylethyl esterified, methyl amidated, etc.); and the like. These compounds can be produced from compound (I) by a method known *per se.*

The prodrug of compound (I) or a salt thereof may be a compound which is converted to compound (I) under physiological conditions as described in "Pharmaceutical Research and Development", Vol. 7 (Drug Design), pages

163-198 published in 1990 by Hirokawa Publishing Co. (Tokyo, Japan).

The compound (T) has a superior tachykinin receptor antagonistic action, particularly a substance P receptor antagonistic action, besides a suppressive action on promoted vascular permeability of trachea, which is induced by capsaicin. The compound (T) has low toxicity and is safe.

As the NK-2 receptor antagonist to be used concurrently with NK-1 receptor antagonist, piperidine derivatives such as GR159897, GR149861, SR48968 (saredutant), SR144190, YM35375, YM38336, ZD7944, L-743986, MDL105212A, ZD6021, MDL105172A, SCH205528, SCH62373, R-113281 and the like, perhydroisoindole derivatives such as RPR-106145 and the like, quinoline derivatives such as SB-414240 and the like, pyrrolopyrimidine derivatives such as ZM-253270 and the like, pseudopeptide derivatives such as MEN11420 (nepadutant), SCH217048, L-659877, PD-147714 (CAM-2291), MEN10376, S16474 and the like, as well as GR100679, DNK333, GR94800, UK-224671, MEN10376, salts thereof and the like.

Of these, MDL105172A, SCH205528, SCH62373, R-113281, ZD6021, S16474, DNK333 and the like are compounds ($NK_1$-$NK_2$ dual antagonists) concurrently having an NK-1 receptor antagonist action and an NK-2 receptor antagonist action. Accordingly, when an $NK_1$-$NK_2$ dual antagonist is to be used for the combination drug of the present invention, it may be used in combination with an NK-1 receptor antagonist, an NK-2 receptor antagonist or an anti-cholinergic drug, or an $NK_1$-$NK_2$ dual antagonist may be used alone.

Examples of the anti-cholinergic drug include atropine, scopolamine, homatropine, tropicamide, cyclopentolate, scopolamine butylbromide, propantheline bromide, methylbenactyzium bromide, mepenzolate bromide, flavoxate, pirenzepine, ipratropium bromide, trihexyphenidyl, oxybutynin, propiverine, darifenacin, tolterodine, temiverine, trospium chloride or a salt thereof (e.g., atropine sulfate, scopolamine hydrobromide, homatropine hydrobromide, cyclopentolate hydrochloride, flavoxate hydrochloride, pirenzepine hydrochloride, trihexyphenidyl hydrochloride, oxybutynin chloride, tolterodine tartrate and the like) and the like. Particularly preferably, oxybutynin, propiverine, darifenacin, tolterodine, temiverine, trospium chloride or a salt thereof (e.g., oxybutynin chloride, tolterodine tartrate, etc.).are used. As the drug that can be used concurrently with compound (T), acetylcholine esterase inhibitors (e.g., distigmine etc.) are preferable.

The combination drug of the present invention is useful as a prophylactic or therapeutic drug of the disease relating to the tachykinin receptor antagonistic action. Specifically, it is useful as a safe prophylactic or therapeutic drug of diseases such as inflammation or allergic diseases (e.g., atopy, dermatitis, herpes, psoriasis, asthma, chronic obstructive pulmonary disease, bronchitis, expectoration, rhinitis, rheumatoid arthritis, osteoarthritis, osteoporosis, multiple sclerosis, conjunctivitis, cystitis and the like), pain, migraine, neuralgia, itch, cough, HIV infectious diseases, further, central nervous system diseases [e.g., Schizophrenia, Parkinson's syndrome, psychosomatic disease, dementia (e.g., Alzheimer's disease and the like) and the like], gastrointestinal diseases [e.g., irritable bowel syndrome, ulcerative colitis, Crohn's disease, aberration (e.g., gastritis, gastric ulcer and the like) caused by urease positive helical Gram negative bacterium (e.g., Helicobacter pylori and the like), and the like], emesis, aberration of miction (e.g., urinary frequency, urinary incontinence and the like), cardiovascular diseases (e.g., angina pectoris, hypertension, cardiac failure, thrombosisand the like) and immunologic aberration and the like in a mammal (e.g., mouse, rat, hamster, rabbit, cat, dog, bovine, sheep, monkey, human and the like). Particularly, the combination drug of the present invention is useful as a tachykinin receptor antagonist or an improving agent or therapeutic drug of aberration of miction such as urinary frequency, urinary incontinence and the like.

In addition, the combination drug of the present invention is useful as a prophylactic or therapeutic agent of diseases such as depression, anxiety neurosis, manic depressive psychosis, autonomic imbalance and the like.

The combination drug of the present invention includes any of (1) a pharmaceutical composition containing two components (NK-1 receptor antagonist and NK-2 receptor antagonist, NK-1 receptor antagonist and anti-cholinergic drug) or three components (NK-1 receptor antagonist, NK-2 receptor antagonist and anti-cholinergic drug) and (2) two components or three components separately prepared. In addition, the combination drug of the present invention includes a pharmaceutical composition for the prophylaxis or treatment of urinary frequency or urinary incontinence, which contains only one component of an $NK_1$-$NK_2$ dual antagonist.

As the combination drug of the present invention, each active ingredient (NK-1 receptor antagonist, NK-2 receptor antagonist, anti-cholinergic drug) can be administered orally or parenterally, separately or simultaneously, as it is or upon admixing with a pharmacologically acceptable carrier and the like, in the form of, for example, a solid preparation such as powder, granule, tablet, capsule and the like, a liquid such as syrup, emulsion, injection (including subcutaneous injection, intravenous injection, intramuscular injection, intravenous infusion) and the like, sublingual tablet, buccal, troche, a sustained release preparation such as microcapsule and the like, an oral cavity rapid disintegrator or a suppository. Moreover, it can be formulated into a transdermal preparation such as patch, pap, ointment (including cream), plaster, pate, lotion, liquid, suspension, emulsion, propellant and the like.

As the above-mentioned pharmacologically acceptable carrier, there are mentioned various conventional, organic or inorganic carriers as a material for the preparation. Examples thereof include excipients, lubricants, binders and disintegrators for solid preparations; and solvents, solubilizing aids, suspending agents, isotonic agents, buffers

and soothing agents for liquid preparations. Where necessary, conventional additives such as antiseptics, antioxidants, coloring agents, sweeteners and the like can be used.

As preferable examples of the above-mentioned excipient, there are mentioned, for example, lactose, sucrose, D-mannitol, starch, crystalline cellulose, light anhydrous silicic acid, calcium carbonate, calcium phosphate and the like.

As preferable examples of the above-mentioned lubricant, there are mentioned, for example, stearic acid, magnesium stearate, calcium stearate, talc, colloidal silica and the like.

As preferable examples of the above-mentioned binder, there are mentioned, for example, crystalline cellulose, sucrose, D-mannitol, dextrin, hydroxypropylcellulose, hydroxypropylmethylcellulose, polyvinylpyrrolidone, acacia, gelatin and the like.

As preferable examples of the above-mentioned disintegrator, there are mentioned, for example, starch, carboxymethylcellulose,carboxymethylcellulose calcium, croscarmellose sodium, sodium carboxymethyl starch and the like.

As preferable examples of the above-mentioned solvent, there are mentioned, for example, injectable water, physiological saline, alcohol, propylene glycol, Macrogol, sesame oil, corn oil and the like.

As preferable examples of the above-mentioned solubilizing aid, there are mentioned, for example, polyethylene glycol, propylene glycol, D-mannitol, benzyl benzoate, ethanol, tris-aminomethane, cholesterol, triethanolamine, sodium carbonate, sodium citrate and the like.

As preferable examples of the above-mentioned suspending agent, there are mentioned, for example, surfactants such as stearyl triethanolamine, sodium lauryl sulfate, lauryl aminopropionic acid, lecithin, benzalkonium chloride, benzethonium chloride, glyceryl monostearate and the like; hydrophilic polymers such as polyvinyl alcohol, polyvinylpyrrolidone, sodium carboxymethylcellulose, methylcellulose, hydroxymethylcellulose, hydroxyethylcellulose, hydroxypropylcellulose and the like.

As preferable examples of the above-mentioned isotonicity agent, there are mentioned, for example, sodium chloride, glycerine, D-mannitol and the like.

As preferable examples of the above-mentioned buffer, there are mentioned, for example, buffers such as phosphate, acetate, carbonate, citrate and the like.

As preferable examples of the above-mentioned soothing agent, there are mentioned, for example, benzyl alcohol and the like.

As preferable examples of the above-mentioned antiseptic, there are mentioned, for example, p-oxybenzoates, chlorobutanol, benzyl alcohol, phenethyl alcohol, dehydroacetic acid, sorbic acid and the like.

As preferable examples of the above-mentioned antioxidant, there are mentioned, for example, sulfite, ascorbic acid and the like.

As preferable examples of the above-mentioned coloring agent, there are mentioned, for example, water-soluble food coal-tar dye, water-insoluble lake dye, natural dye (e.g., β-carotene, chlorophyll, red iron oxide etc.) and the like.

As preferable examples of the above-mentioned sweetening agent, there are mentioned, for example, saccharin sodium, dipotassium glycyrrhizinate, aspartam, stevia and the like.

These preparations can be produced a method known *per se*, which is generally used for a preparation step.

For example, each active ingredient of the combination drug of the present invention can be prepared into an aqueous injection together with a dispersant (e.g., Tween 80 (ATLASPOWDER USA), HCO60 (NIKKO CHEMICALS), polyethylene glycol, carboxymethylcellulose, sodium arginate, hydroxypropylmethylcellulose, dextrin etc.), a stabilizer (e.g., ascorbic acid, sodium pyrosulfite etc.), a surfactant (e.g., polysorbate 80, Macrogol etc.), a solubilizer (e.g., glycerine, ethanol etc.), a buffering agent (e.g., phosphoric acid, alkali metal salt thereof, citric acid, alkali metal salt thereof etc.), an isotonicity agent (e.g., sodium chloride, potassium chloride, mannitol, sorbitol, glucose etc.), a pH adjusting agent (hydrochloric acid, sodium hydroxide etc.), a preservative (ethyl p-hydroxybenzoate, benzoic acid, methylparaben, propylparaben, benzyl alcohol etc.), a solubilizer (e.g., conc. glycerine, meglumine etc.), a solubilizing aid (e.g., propylene glycol, sucrose etc.), a soothing agent (e.g., glucose, benzyl alcohol etc.) and the like, or into an oil-based injection by dissolving, suspending or emulsifying in a vegetable oil (e.g., olive oil, sesame oil, cottonseed oil, corn oil etc.) or a solubilizing aid such as propylene glycol etc., and used as an injection.

An oral formulation can be produced by a method known *per se* by admixing each active ingredient of the combination drug of the present invention with an excipient (e.g., lactose, sucrose, starch and the like), a disintegrant (e.g., starch, calcium carbonate and the like), a binder (e.g., starch, acacia, carboxymethyl cellulose, polyvinyl pyrrolidone, hydroxypropyl cellulose and the like) or a lubricant (e.g., talc, magnesium stearate, polyethylene glycol 6000 and the like) and compressing the mixture, optionally followed by a coating process known per se for the purpose of masking a taste, forming an enteric coat, or achieving a sustained release. Such coating may, for example, be hydroxypropylmethyl cellulose, ethyl cellulose, hydroxymethyl cellulose, hydroxypropyl cellulose, polyoxyethylene glycol, Tween 80, Pluronic F68, cellulose acetate phthalate, hydroxypropylmethyl cellulose phthalate, hydroxymethyl cellulose

acetate succinate, Eudragid (ROHME, Germany, a copolymer of methacrylic acid and acrylic acid), a dye (e.g., red iron oxide, titanium oxide etc.) and the like. The preparation for oral administration may be either a rapid release preparation or a sustained release preparation.

For example, a suppository can be produced by making each active ingredient of the combination drug of the present invention into an oily or aqueous solid, semisolid or liquid composition. Examples of the oily base to be used for such a composition include glyceride of higher fatty acid (e.g., cacao butter, Witepsol (Dynamit Nobel, Germany) etc.), medium fatty acid (e.g., migliol (Dynamit Nobel, Germany) etc.), vegetable oil (e.g., sesame oil, soybean oil, cottonseed oil etc.) and the like. Examples of the aqueous base include polyethylene glycols and propylene glycol, and examples of the aqueous gel base include natural gums, cellulose derivative, vinyl polymer, acrylate polymer and the like

Examples of the above-mentioned sustained release preparation include sustained release microcapsule and the like.

A sustained release microcapsule can be prepared by a method known *per se.* For example, a sustained release preparation shown in the following [2] is preferably formed and administered.

The combination drug of the present invention is preferably formed into a preparation for oral administration such as a solid preparation (e.g., powder, granule, tablet, capsule) and the like, or a preparation for rectal administration such as a suppository and the like. Particularly, a preparation for oral administration is preferable.

The NK-1 receptor antagonist, NK-2 receptor antagonist and anti-cholinergic drug can be prepared into the above-mentioned dosage form according to the kind of the drug.

In the following, [1] injection and preparation thereof, [2] sustained release preparation or rapid release preparation and preparation thereof, [3] sublingual tablet, buccal or oral cavity rapid disintegrator and preparation thereof of the combination drug of the present invention are concretely shown.

[1] Injection and preparation thereof

An injection containing each active ingredient of the combination drug of the present invention dissolved in water is preferable. The injection may contain benzoate and/or salicylate.

The injection is obtained by dissolving each active ingredient of the combination drug of the present invention, as well as, where desired, benzoate and/or salicylate in water.

The salt of the above-mentioned benzoic acid and salicylic acid includes, for example, alkali metal salts such as sodium, potassium and the like, alkaline earth metal salts such as calcium, magnesium and the like, ammonium salt, meglumine salt, and organic acid salt such as trometamol and the like, and the like.

The concentration of each active ingredient of the combination drug of the present invention in the injection is about 0.5 - 50 w/v%, preferably about 3 - 20 w/v%. The concentration of the benzoate and/or salicylate is preferably about 0.5 - 50 w/v%, more preferably about 3- 20 w/v%.

This agent may contain additives generally used for injections, such as a stabilizer (e.g., ascorbic acid, sodium pyrosulfite etc.), a surfactant (e.g., polysorbate 80, Macrogol etc.), a solubilizer (e.g., glycerine, ethanol etc.), a buffering agent (e.g., phosphoric acid, alkali metal salt thereof, citric acid, alkali metal salt thereof etc.), an isotonicityagent (e.g., sodium chloride, potassium chloride etc.), a dispersing agent (e.g., hydroxypropylmethylcellulose, dextrin), a pH adjusting agent (hydrochloric acid, sodium hydroxide etc.), a preservative (ethyl p-hydroxybenzoate, benzoic acid etc.), a solubilizer (e.g., conc. glycerine, meglumine etc.), a solubilizing aid (e.g., propylene glycol, sucrose etc.), a soothing agent (e.g., glucose, benzyl alcohol etc.) and the like as appropriate. These additives are added in a proportion generally employed for injections.

The injection is preferably adjusted to pH 2 - 12, preferably: 2.5 - 8.0, by the use of a pH adjusting agent.

The injection can be obtained by dissolving both of each active ingredient of the combination drug of the present invention and, where desired, benzoate and/or salicylate, and where necessary, the above-mentioned additives in water. These may be dissolved in any order in a suitable manner as in conventional production of injections.

The injectable aqueous solution is preferably heated and, in the same manner as with conventional injections, subjected to, for example, sterilization by filtration, high pressure sterilization by heating and the like to provide an injection.

The injectable aqueous solution is preferably subjected to high pressure sterilization by heating at, for example, 100°C - 121°C for 5 min - 30 min.

It may be prepared into an antibacterial solution, so that it can be used as a preparation for plural subdivided administrations.

[2] Sustained release preparation or rapid release preparation and preparation thereof

A sustained release preparation wherein a core containing each active ingredient of the combination drug of

the present invention is covered on demand with a film forming agent, such as a water-insoluble material, a swellable polymer and the like, is preferable. For example, a sustained release preparation for oral administration once a day is preferable.

The water-insoluble material to be used for the film forming agent is, for example, cellulose ethers such as ethylcellulose, butylcellulose and the like; cellulose esters such as cellulose acetate, cellulose propionate and the like; polyvinyl esters such as poly(vinyl acetate), poly(vinyl butyrate) and the like; acrylic acid/methacrylic acid copolymer, methyl methacrylate copolymer, ethoxyethyl methacrylate/cinnamoethyl methacrylate/aminoalkyl methacrylate copolymer, polyacrylic acid, polymethacrylic acid, methacrylic acid alkylamide copolymer, poly(methyl methacrylate), polymethacrylate, polymethacryl amide, aminoalkyl methacrylate copolymer, poly(methacrylic anhydride) and glycidyl methacrylate copolymer, particularly acrylic polymers such as Eudragits (Rohm Pharma) such as Eudragit RS-100, RL-100, RS-30D, RL-30D, RL-PO, RS-PO (ethyl acrylate-methyl methacrylate-trimethyl chloride methacrylate-ammonium ethyl copolymer), Eudragit NE-30D (methyl methacrylate-ethyl acrylate copolymer) and the like, and the like; hydrogenated oils such as hydrogenated castor oil (e.g., Lubri wax (Freund Inc.) and the like) and the like; waxes such as carnauba wax, fatty acid glycerine ester, paraffin and the like; polyglycerine fatty acid ester and the like.

As the swellable polymer, a polymer having an acidic dissociable group, which shows pH-dependent swelling, is preferable, and a polymer having an acidic dissociable group, which shows less swelling in an acidic range, such as in the stomach, but greater swelling in a neutral range, such as in the small intestine and large intestine, is preferable.

Examples of the polymer having an acidic dissociable group, which shows pH-dependent swelling, include crosslinking type polyacrylic acid polymers such as Carbomer 934P, 940, 941, 974P, 980, 1342 and the like, polycarbophil, calcium polycarbophil (all mentioned above are the product of BF Goodrich), HI-BIS-WAKO 103, 104, 105, 304 (all being products of Waco Pure Chemicals Industries, Ltd.) and the like.

The film forming agent to be used for the sustained release preparation may further contain a hydrophilic material.

Examples of the hydrophilic material include polysaccharides optionally having a sulfuric acid group such as pullulan, dextrin, alkali metal salt of alginic acid and the like; polysaccharides having a hydroxy alkyl group or a carboxy alkyl group such as hydroxypropylcellulose, hydroxypropylmethylcellulose, sodium carboxymethylcellulose and the like; methylcellulose, polyvinylpyrrolidone, polyvinyl alcohol, polyethylene glycol and the like.

The content of the water-insoluble material of the film forming agent for a sustained release preparation is about 30 - about 90% (w/w), preferably about 35 - about,80% (w/w), more preferably about 40 - about 75% (w/w), and the content of the swellable polymer is about 3 - about 30% (w/w), preferably about 3 - about 15% (w/w). The film forming agent may further contain a hydrophilic material, in which case the content of the hydrophilic material for film forming agent is not more than about 50% (w/w), preferably about 5 - about 40% (w/w), more preferably about 5 - about 35% (w/w). As used herein, the above-mentioned % (w/w) is a weight percentage relative to the film forming agent composition wherein the solvent (e.g., water, lower alcohol such as methanol, ethanol and the like) has been removed from the film forming liquid agent.

A sustained release preparation is produced by preparing a core containing a drug as exemplarily mentioned below, and coating the resulting core with a film forming liquid agent prepared by dissolving by heating or dissolving or dispersing in a solvent a water-insoluble material, a swellable polymer and the like.

I. Preparation of core containing a drug

The form of the core containing a drug (hereinafter sometimes simply referred to as a core) to be coated with a film forming agent is not particularly limited, but it is preferably formed into particles such as granules, fine granules and the like.

When the core is made of granules or fine granules, the average particle size thereof is preferably about 150 - about 2,000 μm, more preferably about 500 - about 1,400 μm.

The core can be prepared by a typical production method. For example, a drug is mixed with suitable excipients, binders, disintegrators, lubricants, aggregation preventives, gliding agents, stabilizers and the like, and subjected to wet extrusion granulation, fluidized bed granulation and the like.

The drug content of the core is about 0.5 - about 95% (w/w), preferably about 5.0 - about 80% (w/w), more preferably about 30 - about 70% (w/w).

Examples of the excipient to be contained in the core include saccharides such as sucrose, lactose, mannitol, glucose and the like, starch, crystalline cellulose, calcium phosphate, cornstarch and the like. Of these, crystalline cellulose and corn starch are preferable.

Examples of the binder include polyvinyl alcohol, hydroxypropylcellulose, polyethylene glycol, polyvinylpyrrolidone, Pluronic F68, acacia, gelatin, starch and the like. Examples of the disintegrator include carboxymethylcellulose calcium (ECG505), croscarmellose sodium (Ac-Di-Sol), crosslinked polyvinylpyrrolidone (Crospovidone), low substituted hydroxypropylcellulose (L-HPC) and the like. Of these, hydroxypropylcellulose, polyvinylpyrrolidone and low substituted hydroxypropylcellulose are preferable. Examples of the lubricant and coagulation preventive include talc, magnesium stearate and inorganic salts thereof, and examples of the lubricant include polyethylene glycol and the like. Examples of the stabilizer include acids such as tartaric acid, citric acid, succinic acid, fumaric acid, maleic acid and the like.

The core can be also prepared by, besides the above-mentioned production methods, for example, rolling granulation wherein a drug or a mixture of a drug and an excipient; a lubricant and the like is added by small portions while spraying a binder dissolved in a suitable solvent such as water, lower alcohol (e.g., methanol, ethanol and the like) and the like on an inert carrier particles to be the center of the core, a pan coating method, a fluidized bed coating method or a melt granulating method. Examples of the inert carrier particle include those prepared from sucrose, lactose, starch, crystalline cellulose and waxes, which preferably have an average particle size of about 100 µm about 1,500 µm.

To separate the drug contained in the core from the film forming agent, the surface of the core may be coated with a protective agent. Examples of the protective agent include the aforementioned hydrophilic material, water-insoluble material and the like. As the protective agent, preferably polyethylene glycol, polysaccharides having a hydroxyalkyl group or a carboxy alkyl group, more preferably hydroxypropylmethylcellulose and hydroxypropylcellulose are used. The protective agent may contain, as a stabilizer, an acid such as tartaric acid, citric acid, succinic acid, fumaric acid, maleic acid and the like, and a lubricant such as talc and the like. When the protective agent is used, the amount to be coated is about 1 - about 15% (w/w), preferably about 1 - about 10% (w/w), more preferably about 2 - about 8% (w/w), relative to the core.

The protective agent can be coated by a typical coating method. Specifically, the protective agent is, for example, spray-coated to the core by a fluidized bed coating method, a pan coating method, and the like.

II. Coating of core with a film forming agent

The core obtained in the aforementioned I is coated with a film forming liquid agent prepared by dissolving by heating or dissolving or dispersing in a solvent the aforementioned water-insoluble material, a pH-dependent swellable polymer, and a hydrophilic material to provide a sustained release preparation.

For coating a core with a film forming liquid agent, for example, a spray coatingmethod and the like can be employed.

The composition ratio of the water-insoluble material, swellable polymer or hydrophilic material in the film forming liquid agent is suitably determined such that each component, of the coating film meets the aforementioned content.

The coating amount of the film forming agentis about 1 - about 90% (w/w), preferably about 5 - about 50% (w/w), more preferably about 5 - 35% (w/w), relative to the core (exclusive of the coating amount of protective agent).

As the solvent for the film forming liquid agent, water or organic solvents can be used alone or in a mixture of the both. The mixing ratio (water/organic solvent: weight ratio) of water and the organic solvent in the mixture can vary within the range of about 1 - about 100%, which is preferably about 1 - about 30%. The organic solvent is not subject to any particular limitation as long as it dissolves the water-insoluble material. For example, lower alcohols such as methyl alcohol, ethyl alcohol, isopropyl alcohol, n-butyl alcohol and the like, lower alkanone such as acetone and the like, acetonitrile, chloroform, methylene chloride and the like are used. Of these, lower alcohol is preferable, and ethyl alcohol and isopropyl alcohol are particularly preferable. Water and a mixture of water and an organic solvent are preferably used as a solvent of the film forming agent. Where necessary, the film forming liquid agent may contain an acid such as tartaric acid, citric acid, succinic acid, fumaric acid, maleic acid and the like for the stabilization of the film forming liquid agent.

When spray coating is employed, the method follows a conventional coating method, which specifically includes spray coating the core with a film forming liquid agent by, for example, a fluidized bed coating method, a pan coating method and the like. Where necessary, talc, titanium oxide, magnesium stearate, calcium stearate, light anhydrous silicic acid and the like may be added as a lubricant, and glycerine fatty acid ester, hydrogenated castor oil, triethyl citrate, cetyl alcohol, stearyl alcohol and the like may be added as a plasticizer.

After coating with a film forming agent, an antistatic agent such as talc and the like may be added as necessary.

A rapid release preparation may be,a liquid (solution, suspension, emulsion and the like) or a solid (particle, pill, tablet and the like). An agent for oral administration, and an agent for parenteral administration, such as injection and the like, are used, with preference given to an agent for oral administration.

A rapid release preparation may generally contain, in addition to the drug, which is an active ingredient, carriers, additives and excipients (hereinafter also generally referred to as excipient) conventionally used in the field of preparation. The excipient is not subject to any particular limitation as long as it is conventionally employed as an excipient in the field of pharmaceutical preparation. For example, the excipient for the oral solid preparation includes lactose, starch, corn starch, crystalline cellulose (Asahi Kasei Corporation, Avicel PH101 and the like), powder sugar,

granulated sugar, mannitol, light anhydrous silicic acid, magnesium carbonate, calcium carbonate, L-cysteine and the like, preferably corn starch and mannitol and the like. These excipients may be used alone or in combination. The content of the excipient is, for example, about 4.5 - about 99.4 w/w%, preferably about 20 - about 98.5 w/w%, more preferably about 30 - about 97 w/w%, of the total amount of the rapid release preparation.

The drug content of the rapid release preparation is appropriately determined from the range of about 0.5 - about 95%, preferably about 1 - about 60%, of the total amount of the rapid release preparation.

When the rapid release preparation is an oral solid preparation, it generally contains a disintegrator in addition to the above-mentioned components. Examples of the disintegrator include those capable of collapsing granules by, for example, water absorption, swelling or forming a channel between the active ingredient and an excipient constituting the core, upon contact with water. For example, calcium carboxymethylcellulose (Gotoku Pharmaceutical Co., Ltd., ECG-505), croscarmellose sodium (e.g., Asahi Kasei Corporation, acjizol), Crospovidone (e.g., colidone CL, BASF), low substituted hydroxypropylcellulose (Shin-Etsu Chemical Co., Ltd.), carboxymethyl starch (Matsutani Chemical Industry Co., Ltd.), sodium carboxymethyl starch (Kimura Sangyo, exprotab), partially α starch (PCS, Asahi Kasei Corporation) and the like. These disintegrators can be used alone or in combination. The amount of the disintegrator is appropriately determined depending on the kind of the drug to be used and amount thereof, design of the release preparation and the like. It is generally about 0.05 - about 30 w/w%, preferably about 0.5 - about 15 w/w%, relative to the total amount of the rapid release preparation.

When the rapid release preparation is an oral solid preparation, the oral solid preparation may further contain, in addition to the above-mentioned composition, typical additives used for solid preparation on demand. Examples of the additive include a binder (e.g., sucrose, gelatin, acacia powder, methylcellulose, hydroxypropylcellulose, hydroxypropylmethylcellulose, carboxymethylcellulose, polyvinylpyrrolidone, Pullulan, dextrin etc.), a lubricant (e.g., polyethylene glycol, magnesium stearate, talc, light anhydrous silicic acid (e.g., Aerosil (Nippon Aerosil)), a surfactant (e.g., anionic surfactant such as sodium alkylsulfate etc., non-ionic surfactant such as polyoxyethylene fatty acid ester and polyoxyethylenesorbitan fatty acid ester, polyoxyethylene castor oil derivative etc., and the like), a coloring agent (e.g., tar color, caramel, red iron oxide, titanium oxide, riboflavins), where necessary, a corrigent (e.g., a sweetener, flavor etc.), an absorbent, an antiseptic, a moistening agent, an antistatic agent and the like. As the stabilizer, an organic acid such as tartaric acid, citric acid, succinic acid, fumaric acid and the like may be added.

Examples of the above-mentioned binder preferably include hydroxypropylcellulose, polyethylene glycol, polyvinylpyrrolidone and the like.

The rapid release preparation can be prepared based on the conventional preparation method, by mixing each of the aforementioned components, and where necessary, further kneading and forming. The above-mentioned mixing can be performed by a conventional method, such as mixing, kneading and the like. Specifically, for example, when a rapid release preparation is formed into particles, a vertical granulator, a universal kneader (HATA Tekkohjo), a fluidized bed granulator FD-5S (Powrex Corporation) and the like are used for mixing, which is followed by granulating by wet extrusion granulation, fluidized bed granulation and the like, to give the preparation, as in the preparation of the core of the aforementioned sustained release preparation.

The rapid release preparation and the sustained release preparation thus obtained may be used as they are. Alternatively, after suitable separate preparation along with an excipient for a preparation and the like according to a conventional method, they may be administered simultaneously or at optional administration intervals. Alternatively, they may be each prepared into a single preparation for oral administration (e.g., granule, fine granule, tablet, capsule and the like) as they are or together with excipient for preparation and the like as appropriate. The both preparations are converted to granules or fine granules and filled in a single capsule and the like to give a preparation for oral administration.

[3] A sublingual tablet, buccal or oral cavity rapid disintegrator and preparation thereof

When the combination drug of the present invention is formed as a sublingual tablet, buccal preparation or oral cavity rapid disintegrator, it may be a solid preparation such as tablet and the like or an oral cavity mucous membrane adhesion tablet (film).

As the sublingual tablet, buccal or oral cavity rapid disintegrator, a preparation containing an anti-cholinergic agent or NK-2 receptor antagonist and an excipient is preferable. It may contain auxiliaries such as a lubricant, an isotonic agent, a hydrophilic carrier, a water dispersible polymer, a stabilizer and the like. For easy absorption and enhanced bioavailability, β-cyclodextrin or β-cyclodextrin derivative (e.g., hydroxypropyl-β-cyclodextrin and the like) and the like may be contained.

Examples of the above-mentioned excipient include lactose, sucrose, D-mannitol, starch, crystalline cellulose, light anhydrous silicic acid and the like. Examples of the lubricant include magnesium stearate, calcium stearate, talc, colloidal silica and the like, particularly magnesium stearate and colloidal silica are preferable. Examples of the isotonicity agent include sodium chloride, glucose, fructose, mannitol, sorbitol, lactose, saccharose, glycerine, urea and the

like, particularly mannitol is preferable. Examples of the hydrophilic carrier include swellable hydrophilic carriers such as crystalline cellulose, ethylcellulose, crosslinked polyvinylpyrrolidone, light anhydrous silicic acid, silicic acid, dicalcium phosphate, calcium carbonate and the like, particularly crystalline cellulose (e.g., microcrystalline cellulose and the like) is preferable. Examples of the water dispersible polymer include gum (e.g., gum tragacanth, acacia gum, guar gum), alginate (e.g., sodium alginate), cellulose derivative (e.g., methylcellulose, carboxymethylcellulose, hydroxymethylcellulose, hydroxypropylcellulose, hydroxypropylmethylcellulose), gelatin, soluble starch, polyacrylic acid (e.g., carbomer), polymethacrylic acid, polyvinyl alcohol, polyethylene glycol, polyvinylpyrrolidone, polycarbofil and the like, with preference given to hydroxypropylmethylcellulose, polyacrylic acid, alginate, gelatin, carboxymethylcellulose, polyvinylpyrrolidone, polyethylene glycol and the like. Particularly, hydroxypropylmethylcellulose is preferable. Examples of the stabilizer include cysteine, thiosorbitol, tartaric acid, citric acid, sodium carbonate, ascorbic acid, glycine, sodium sulfite and the like, particularly, citric acid and ascorbic acid are preferable.

The sublingual tablet, buccal and oral cavity rapid disintegrator can be produced by mixing each active ingredient of the combination drug of the present invention and the above-mentioned excipient by a method known *per se*. Where desired, the above-mentioned auxiliaries such as a lubricant, an isotonic agent, a hydrophilic carrier, a water dispersible polymer, a stabilizer, a coloring agent, a sweetener, an antiseptic and the like may be contained. After mixing the above-mentioned components simultaneously or with time staggering, the mixture is compression formed under pressure to give sublingual tablet, buccal or oral cavity rapid disintegrator. To achieve a suitable hardness, a solvent such as water, alcohol and the like is used to moisten or wet as necessary before and after the compression forming. After the forming, the tablets may be dried.

When a mucous membrane adhesion tablet (film) is produced, each active ingredient of the combination drug of the present invention and the above-mentioned water dispersible polymer (preferably, hydroxypropylcellulose, hydroxypropylmethylcellulose), an excipient and the like are dissolved in a solvent such as water and the like, and the obtained solution is cast to give a film. In addition, an additive such as a plasticizer, a stabilizer, an antioxidant, a preservative, a coloring agent, a buffer, a sweetener and the like may be added. To impart suitable elasticity to the film, glycols such as polyethylene glycol, propylene glycol and the like may be added, and to increase adhesion of the film to the oral cavity mucous membrane lining, bioadhesive polymer (e.g., polycarbofil, carbopol) may be added. The casting includes pouring the solution on a non-adhesive surface, spreading the solution in a uniform thickness (preferably about 10 - 1000 micrometers) with a coating tool such as doctor blade and the like and drying the solution to give a film. The film thus formed may be dried at room temperature or under heating and cut into a desired surface area.

Examples of preferable oral cavity rapid disintegrator are a solid rapid diffusing administration agent having a net structure of each active ingredient of the combination drug of the present invention and a water soluble or water diffusable carrier which are inert to each active ingredient of the combination drug of the present invention. The net structure can be obtained by evaporation of a solvent from the solid composition consisting of a solution obtained by dissolving each active ingredient of the combination drug of the present invention in a suitable solvent.

The oral cavity rapid disintegrator preferably contains, in addition to each active ingredient of the combination drug of the present invention, a matrix forming agent and a secondary component.

Examples of the matrix forming agent include animal proteins or vegetable proteins such as gelatins, dextrins, soybeans, wheat, psyllium seed protein and the like; rubber substances such as acacia, guar gum, agar, xanthan and the like; polysaccharides; alginic acids; carboxymethylcelluloses; carrageenans; dextrans; pectins; synthetic polymers such as polyvinylpyrrolidone and the like; a material derived from a gelatin-acacia complex and the like. In addition, saccharides such as mannitol, dextrose, lactose, galactose, trehalose and the like; cyclic saccharides such as cyclodextrin and the like; inorganic salts such as sodium phosphate, sodium chloride, aluminum silicate and the like; amino acid having 2 to 12 carbon atoms such as glycine, L-alanine, L-aspartic acid, L-glutamine acid, L-hydroxyproline, L-isoleucine, L-leucine, L-phenylalanine and the like are exemplified.

It is possible to introduce one or more matrix forming agents into a solution or suspension before preparation into a solid. Such matrix forming agent may exist with a surfactant or without a surfactant. The matrix forming agent can form a matrix, and also can help maintain diffusion state of NK-1 receptor antagonist, anti-cholinergic drug and NK-2 receptor in a solution or suspension.

The composition may contain a secondary component such as a preservative, an antioxidant, a surfactant, a thickener, a coloring agent, a pH adjusting agent, a flavor, a sweetener, a taste masking reagent and the like. As a suitable preservative, parabens and sorbic acid can be mentioned. As a suitable antioxidant, sulfite and ascorbic acid can be mentioned. As a suitable surfactant, polysorbate 80 and macrogol can be mentioned. As a suitable thickener, natural gums, cellulose derivatives can be mentioned. Examples of a suitable coloring agent include red, black and yellow ferric oxides and FD&C dyes of Ellis & Everard, such as FD&C blue NO. 2, FD&C red No. 40 and the like. A suitable flavor contains mint, raspberry, licorice, orange, lemon, grapefruit, caramel, vanilla, cherry, grape flavor and a combination of these. Suitable pH adjusting agent includes citric acid, tartaric acid, phosphoric acid, hydrochloric acid and maleic acid. Suitable sweetener includes aspartame, acesulfame K, thaumatin and the like. Suitable taste masking agent includes sodium bicarbonate, ion exchange resin, cyclodextrin inclusion compound, adsorbent substance and microcapsuled apomorphine.

As the preparation, one containing each active ingredient of the combination drug of the present invention generally in a proportion of about 0.1 -about 50 wt%, preferably about 0.1 - about 30 wt%, which is capable of dissolving 90% or more of each active ingredient of the combination drug of the present invention (in water) for about 1 min - about 60 min, preferably about 1 min - about 15 min, more preferably about 2 min - about 5 min, such as the above-mentioned sublingual tablet, buccal and the like, and an oral cavity rapid disintegrator that disintegrates within 1 - 60 sec, preferably 1 - 30 sec, more preferably 1 - 10 sec, after being placed in an oral cavity, are preferable.

The content of the above-mentioned excipient in the whole preparation is about 10 - about 99 wt%, preferably about 30 - about 90 wt%. The content of the β-cyclodextrin or β-cyclodextrin derivative relative to the whole preparation is 0 - about 30 wt%. The content of the lubricant relative to the whole preparation is about 0.01 - about 10 wt%, preferably about 1 - about 5 wt%. The content of the isotonicity agent relative to the whole preparation is about 0.1 - about 90 wt%, preferably about 10 - about 70 wt%. The content of the hydrophilic carrier relative to the whole preparation is about 0.1 - about 50 wt%, preferably about 10 - about 30 wt%. The content of the water dispersible polymer relative to the whole preparation is about 0.1 - about 30 wt%, preferably about 10-about 25 wt%. The content of the stabilizer relative to the whole preparation is about 0.1 - about 10 wt%, preferably about 1 - about 5 wt%. The above-mentioned preparation may contain additives such as a coloring agent, a sweetener, an antiseptic and the like as necessary.

The daily dose of the combination drug of the present invention varies depending on the level of symptom, age, sex, body weight and difference in sensitivity of the subject of administration, time and interval of administration, property, dispensation and kind of pharmaceutical preparation, kind of active ingredient and the like, and is free of particular limitation. The dose of the active ingredient of the combination drug of the present invention is not particularly limited as long as the side effect is at a non-problematic level. It is generally about 0.005 - about 100 mg, preferably about 0.05 - about 50 mg, more preferably about 0.2 - about 30 mg, per 1 kg body weight of a mammal by oral administration, which is generally administered 1 to 3 times a day in divided doses.

The dose of the active ingredient of the combination drug of the present invention is not particularly limited as long as the side effect is at a non-problematic level. The daily dose of each active ingredient of the combination drug of the present invention varies depending onthe level of symptom, age, sex, body weight and difference in sensitivity of the subject of administration, time and interval of administration, property, dispensation and kind of pharmaceutical preparation, kind of active ingredient and the like, and is free of particular limitation. The amount of the drug is, for example, generally about 0.001-2000 mg, preferably about 0.01-500 mg, more preferably about 0.1-100 mg, per 1 kg body weight of a mammal by oral administration, which is generally administered 1 to 4 times a day in divided doses.

For administration of the combination drug of the present invention, two or three components may be administered at the same time, or after administering one component, other components may be administered. Alternatively, two components are formed into a single preparation and one component as a different preparation, and they may be separately or simultaneously administered. For administration with a time lag, the time lag varies depending on the active ingredient to be administered, the dosage form and the administration method. For example, when an anti-cholinergic drug or NK-2 receptor antagonist is to be administered first, the NK-1 receptor antagonist is administered in 1 min. - 3 days, preferably 10 min. - 1 day, more preferably 15 min. - 1 hr., after administration of the anti-cholinergic drug or NK-2 receptor antagonist. When an NK-1 receptor antagonist is to be administered first, the anti-cholinergic drug or NK-2 receptor antagonist is administered in 1 min. - 1 day, preferably 10 min. - 6 hr., more preferably 15 min. - 1 hr., after administration of the NK-1 receptor antagonist.

As a preferable administration method, for example, about 0.001 - about 200 mg/kg of an anti-cholinergic drug or NK-2 receptor antagonist formed in an oral preparation is orally administered, and about 15 min. later, a daily dose of about 0.005- about 100 mg/kg of compound (T) formed in an oral preparation is orally administered.

In the combination drug of the present invention, the content of compound (T) relative to the entire preparation varies depending on the form of the preparation. It is generally about 0.01-100 wt%, preferably about 0.1-50 wt%, more preferably about 0.5-20 wt%, of the entire preparation.

The site of action of the NK-1 receptor antagonist is considered to be the central nervous system including the spinal cord. In contrast, the site of action of the NK-2 receptor antagonist and anti-cholinergic drug is considered to be the peripheral nervous system. By the concurrent use of drugs having different site of action, the dose thereof can be reduced as compared to a single administration, and a sufficient treatment effect of urinary frequency and urinary incontinence can be achieved with a small dose.

By the concurrent use of an NK-1 receptor antagonist and an anti-cholinergic drug and/or an NK-2 receptor antagonist, the dose thereof can be reduced as compared to a single administration of the anti-cholinergic drug or NK-2 receptor antagonist, and, for example, the side effect that the anti-cholinergic drugs have, such as dry mouth and the like, can be reduced.

Even when not particularly clearly indicated in the present specification, the term, NK-1 receptor antagonist, encompasses NK-1 receptor antagonists, salts thereof and prodrugs thereof, and the term, NK-2 receptor antagonist, encompasses NK-2 receptor antagonists, salts thereof and prodrugs thereof.

The present invention is explained in more detail in the following by referring to Reference Examples, Examples and Experimental Examples. The present invention is not limited in any way by the examples and may be modified within the range that does not deviate from the scope of the present invention.

## Examples

### Reference Example 1

| | |
|---|---|
| (1) Compound No. 3 | 10.0 mg |
| (2) Lactose | 60.0 mg |
| (3) Corn starch | 35.0. mg |
| (4) Hydroxypropylmethylcellulose | 3.0 mg |
| (5) Magnesium stearate | 2.0 mg |

A mixture of 10.0 mg of the compound No. 3, 60.0 mg of lactose and 35.0 mg of corn starch was granulated using 10 wt% aqueous hydroxypropylmethylcellulose solution (0.03 ml, 3.0 mg as hydroxypropylmethylcellulose), dried at 40°C and passed through a sieve. The obtained granules were mixed with magnesium stearate (2.0 mg) and compressed. The obtained naked tablets were sugar-coated with an aqueous suspension of sucrose, titanium dioxide, talc and acacia. The coated tablets were polished with bee wax to give coated tablets.

### Reference Example 2

| | |
|---|---|
| (1) Compound No. 3 | 10.0 mg |
| (2) Lactose | 70.0 mg |
| (3) Corn starch | 50.0 mg |
| (4) Soluble starch | 7.0 mg |
| (5) Magnesium stearate | 3.0 mg |

The compound No. 3 (10.0 mg) and magnesium stearate (3.0 mg) were granulated using 0.07 ml of aqueous solution of soluble starch (7.0 mg as soluble starch), dried and admixed with lactose (70.0 mg) and corn starch (50.0 mg). The mixture was compressed to give tablets.

### Reference Example 3

| | |
|---|---|
| Compound No.3 | 200 mg |
| Sodium benzoate | 200 mg |
| Distilled water for injection total amount | 2 ml |

The compound No. 3 and sodium benzoate are dissolved in distilled water for injection, and prepared aseptically.

### Reference Example 4

| | |
|---|---|
| Oxybutynin chloride | 200 mg |
| Sodium benzoate | 200 mg |
| Distilled water for injection total amount | 2 ml |

Oxybutynin chloride and sodium benzoate are dissolved in distilled water for injection, and prepared aseptically.

**Reference Example 5**

| | |
|---|---|
| Tolterodine tartrate | 200 mg |
| Sodium benzoate | 200 mg |
| Distilled water for injection total amount | 2 ml |

Tolterodine tartrate and sodium benzoate are dissolved in distilled water for injection, and prepared aseptically.

**Reference Example 6**

| | |
|---|---|
| (±) SR48968 Hydrochloride | 200 mg |
| Sodium benzoate | 200 mg |
| Distilled water for injection total amount | 2 ml |

(±)SR48968 Hydrochloride and sodium benzoate are dissolved in distilled water for injection, and prepared aseptically.

**Example 1**

Any preparation from Reference Examples 1-3 and a preparation of Reference Example 4 are separately administered for combined use.

**Example 2**

Any preparation from Reference Examples 1-3 and preparation of Reference Example 5 are separately administered for combined use.

**Example 3**

Any preparation from Reference Examples 1-3 and a preparation of Reference Example 6 are separately administered for combined use.

**Example 4**

Any preparation from Reference Examples 1-3, a preparation of Reference Example 4 and a preparation of Reference Example 6 are separately administered for combined use.

**Experimental Example 1**

The effect afforded by the combined use of compound No. 3 and (±) SR48968 hydrochloride on the suppressive action on pollakiuria and urinary incontinence was shown by the ability to increase bladder capacity of cyclophosphamide-induced pollakiuria rats under a urethane anesthesia. Cyclophosphamide (200 mg/kg) or a solvent was intraperitoneally administered and the rats were subjected to the bladder capacity test the next day. After emptying the intravesical by drawing urine, physiological saline was injected into the intravesical at a constant rate (0.3 ml/min.) to induce miction. This step was repeated, and after confirmation of stable bladder capacity (amount of physiological saline injected until miction was induced), the compound was intravenously injected and its action was examined. The changes in the bladder capacity at 10 min. after administration were measured. The results are shown in Table 1. While the administration of cyclophosphamide decreased the bladder capacity, the combined use of compound No. 3 and (±) SR48968 hydrochloride in a dose that does not increase the bladder capacity when administered solely resulted in an increased bladder capacity, thereby demonstrating the effect by the combined use of the two drugs.

Table 1.

| Effect of combined use of Tachykinin NK-1 receptor antagonist and tachykinin NK-2 receptor antagonist on bladder capacity of guinea pig under urethane anesthesia | | | | |
|---|---|---|---|---|
| Presence or otherwise of Cyclophosphamide administration (200 mg/kg, i.p.) | drug, intravenous administration (dose, mg/kg) | bladder capacity | | |
| | | Before administration (ml) | After administration (ml) | (%) increase |
| - | - | 1.69±0.23 | - | - |
| + | solvent | 1.10±0.37 | 0.94±0.12 | 3.6±14.1 |
| + . | Compound No.3 (0.3) | 1.06±0.14 | 1.05±0.14 | -0.28±5.3 |
| + | SR48968 (10) | 0.99 ± 0.08 | 1.03 ± 0.09 | 3.8 ± 3.3 |
| + | Compound No.3 (0.3) + SR48968 (10) | 1.02 ± 0.14 | 1.62 ± 0.28 | 55.0 ± 10.1 * |

The values shows mean±standard error of 5-7 animals.
* P<0.05 (vs. solvent administration control group, Dunnett's test)

The drug was dissolved in a solvent (N,N-dimethylacetamide-polyethylene glycol 400 (V/V, 1:1)) and intravenously injected. The data are expressed in mean±standard error.

**Experimental Example 2**

The effect afforded by the combined use of compound No. 3 and oxybutynin hydrochloride on the suppressive action on pollakiuria and urinary incontinence was shown by the ability to increase bladder capacity of cyclophosphamide-induced pollakiuria rats under a urethane anesthesia. Cyclophosphamide (200 mg/kg) or a solvent was intraperitoneally administered and the rats were subjected to the bladder capacity test the next day. After emptying the intravesical by drawing urine, physiological saline was injected into the intravesical at a constant rate (0.3 ml/min.) to induce miction. This step was repeated, and after confirmation of stable bladder capacity (amount of physiological saline injected until miction was induced), the compound was intravenously injected and its action was examined. The changes in the bladder capacity at 10 min. after administration were measured. The results are shown in Table 2. While the administration of cyclophosphamide decreased the bladder capacity, the combined use of compound No.3 and oxybutynin hydrochloride in a dose that does not increase the bladder capacity when administered solely resulted in an increased bladder capacity, thereby demonstrating the effect by the combined use of the two drugs.

Table 2.

| Effect of combined use of Tachykinin NK-1 receptor antagonist and oxybutynin chloride (anti-cholinergic drug) on bladder capacity of guinea pig under urethane anesthesia | | | | |
|---|---|---|---|---|
| Presence or otherwise of Cyclophosphamide administration (200 mg/kg, i.p.) | drug, intravenous administration (dose, mg/kg) | bladder capacity | | |
| | | Before administration (ml) | After administration (ml) | (%) increase |
| - | - | 1.58±0.27 | - | - |
| + | solvent | 0.60±0.07 | 0.56±0.08 | -0.7±14.2 |
| + | Compound No.3 (0.3) | 0.66±0.08 | 0.80±0.09 | 28.6±12.4 |
| | oxybutynin chloride | 0.76±0.13 | 0.78±0.12 | 6.9±12.4 |
| + | (1) | | | |
| | Compound No.3 (0.3) | | | |

Table 2. (continued)

| Effect of combined use of Tachykinin NK-1 receptor antagonist and oxybutynin chloride (anti-cholinergic drug) on bladder capacity of guinea pig under urethane anesthesia | | | | |
|---|---|---|---|---|
| Presence or otherwise of Cyclophosphamide administration (200 mg/kg, i.p.) | drug, intravenous administration (dose, mg/kg) | bladder capacity | | |
| | | Before administration (ml) | After administration (ml) | (%) increase |
| + | + oxybutynin chloride (1) | 0.66±0.12 | 1.40±0.22 | 198.7±112.2 * |

The values shows mean±standard error of 5-7 animals.
* P<0.05 (vs. solvent administration control group, Dunnett's test)

The drug was dissolved in a solvent (N,N-dimethylacetamide-polyethylene glycol 400 (V/V, 1:1)) and intravenously injected. The data are expressed in mean±standard error.

**Industrial Applicability**

By the combined use of an NK-1 receptor antagonist (particularly compound (I) or a salt thereof or a prodrug thereof) and an anti-cholinergic drug or NK-2 receptor antagonist,

(1) a superior treatment effect of the diseases such as urinary frequency, urinary incontinence, asthma, chronic obstructive pulmonary disease, rheumatoid arthritis, osteoarthritis, pain, cough, irritable bowel syndrome, emesis, depression, anxiety, manic depressive psychosis, schizophrenia and the like can be exerted,
(2) the dose of an anti-cholinergic drug and an NK-2 receptor antagonist can be reduced as compared to a single administration thereof, and
(3) the side effects that each pharmaceutical agent has can be reduced.

This application is based on a patent application No. 2000-391013 filed in Japan, the contents of which are hereby incorporated by reference.

**Claims**

**1.** A pharmaceutical agent comprising a compound (I) represented by the formula

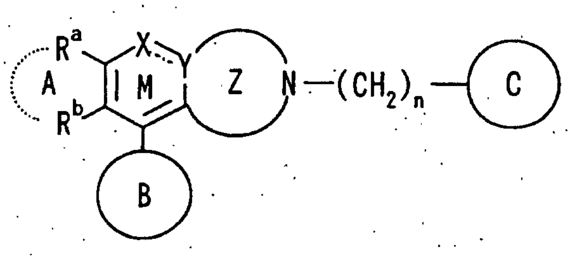

wherein

| | |
|---|---|
| ring M | is a heterocyclic ring having -N=C<, -CO-N< or - CS-N< as a partial structure -X ------ Y < ; |
| $R^a$ and $R^b$ | are bonded to each other to form ring A, or the same or different and each is a hydrogen atom or a substituent on ring M; |
| ring A and ring B | are each a homocyclic or heterocyclic ring optionally having substituents, wherein at least one of them is a heterocyclic ring optionally having substituents; |

ring C is a homocyclic or heterocyclic ring optionally having substituents;
ring Z is an optionally substituted heterocyclic ring containing nitrogen; and
n is an integer of 1 to 6,

or a salt thereof or a prodrug thereof and an NK-2 receptor antagonist in combination.

2. The pharmaceutical agent of claim 1, wherein the compound (I) is

(i) (9S)-7-[3,5-bis(trifluoromethyl)benzyl]-6,7,8,9,10,12-hexahydro-9-methyl-6,12-dioxo-5-phenyl[1,4]diazepino[2,1-g] [1,7]naphthyridine,
(ii) (9s)-7-[3,5-bis(trifluoromethyl)benzyl]-6,7,8,9,10,12-hexahydro-9-methyl-5-(4-methylphenyl)-6,12-dioxo [1,4]diazepino[2,1-g][1,7]naphthyridine,
(iii) (9R)-7-[3,5-bis(trifluoromethyl)benzyl]-6,7,8,9,10,11-hexahydro-9-methyl-6,13-dioxo-5-phenyl-13H-[1,4] diazocino[2,1-g][1,7]naphthyridine,
(iv) (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine,
(v) (9R)-7-(3,5-dimethoxybenzyl)-5-(4-fluorophenyl)-6,7,8,9,10,11-hexahydro-9-methyl-6,13-dioxo-13H-[1,4] diazocino[2,1-g] [1,7]naphthyridine or
(vi)(9R)-7-(3,5-dimethoxybenzyl)-6,7,8,9,10,11-hexahydro-9-methyl-5-(4-methylphenyl)-6,13-dioxo-13H-[1,4]diazocino[2,1-g] [1,7]naphthyridine.

3. The pharmaceutical agent of claim 1, wherein the compound (I) is (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine.

4. The pharmaceutical agent of claim 1, wherein the NK-2 receptor antagonist is a piperidine derivative, a perhydroisoindole derivative, a quinoline derivative, a pyrrolopyrimidine derivative or a pseudopeptide derivative.

5. The pharmaceutical agent of claim 1, which is GR94800, GR159897, MEN10627, MEN11420 (nepadutant), SR144190, SR48968 (saredutant), GR149861, YM35375, YM38336, ZD7944, L-743986, MDL105212A, ZD6021, MDL105172A, SCH205528, SCH62373, R-113281, RPR-106145, SB-414240, ZM-253270, SCH217048, L-659877, PD-147714 (CAM-2291), MEN10376, S16474, GR100679, DNK333, GR94800, UK-224671, MEN10376 or a salt thereof.

6. The pharmaceutical agent of claim 1, which is a prophylactic or therapeutic agent of urinary frequency, urinary incontinence, asthma, chronic obstructive pulmonary disease, rheumatoid arthritis, osteoarthritis, pain, cough, irritable bowel syndrome, emesis, depression, anxiety, manic depressive psychosis or schizophrenia.

7. A pharmaceutical agent comprising an NK-1 receptor antagonist, an NK-2 receptor antagonist and an anti-cholinergic drug in combination.

8. A prophylactic or therapeutic agent of urinary frequency or urinary incontinence, which comprises an NK-1 receptor antagonist and an NK-2 receptor antagonist in combination.

9. A method for the prophylaxis or treatment of urinary frequency, urinary incontinence, asthma, chronic obstructive pulmonary disease, rheumatoid arthritis, osteoarthritis, pain, cough, irritable bowel syndrome, emesis, depression, anxiety, manic depressive psychosis or schizophrenia, which comprises administering, to a mammal, an effective amount of compound (I) represented by the formula

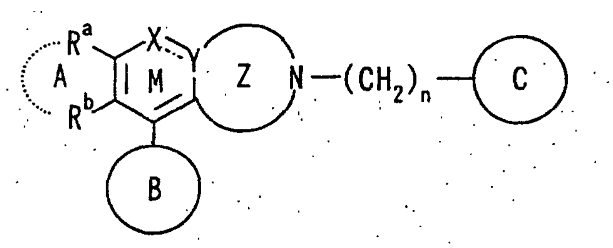

wherein

| | |
|---|---|
| ring M | is a heterocyclic ring having -N=C<, -CO-N< or -CS-N< as a partial structure —X ------ Y < ; |
| $R^a$ and $R^b$ | are bonded to each other to form ring A, or the same or different and each is a hydrogen atom or a substituent on ring M; |
| ring A and ring B | are each a homocyclic or heterocyclic ring optionally having substituents, wherein at least one of them is a heterocyclic ring optionally having substituents; |
| ring C | is a homocyclic or heterocyclic ring optionally having substituents; |
| ring Z | is an optionally substituted heterocyclic ring containing nitrogen; and |
| n | is an integer of 1 to 6, |

or a salt thereof or a prodrug thereof and an effective amount of an NK-2 receptor antagonist in combination.

**10.** Use of compound (I) represented by the formula

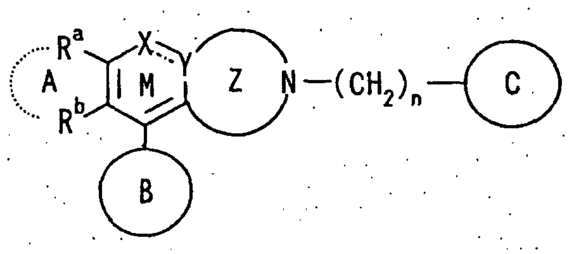

wherein

| | |
|---|---|
| ring M | is a heterocyclic ring having -N=C<, -CO-N< or. -CS-N< as a partial structure - X ------ Y < ; |
| $R^a$ and $R^b$ | are bonded to each other to form ring A, or the same or different and each is a hydrogen atom or a substituent on ring M; |
| ring A and ring B | are each a homocyclic or heterocyclic ring optionally having substituents, wherein at least one of them is a heterocyclic ring optionally having substituents; |
| ring C | is a homocyclic or heterocyclic ring optionally having substituents; |
| ring Z | is an optionally substituted heterocyclic ring containing nitrogen; and |
| n | is an integer of 1 to 6, |

or a salt thereof or a prodrug thereof and an NK-2 receptor antagonist, for the production of a prophylactic or therapeutic agent of urinary frequency, urinary incontinence, asthma, chronic obstructive pulmonary disease, rheumatoid arthritis, osteoarthritis, pain, cough, irritable bowel syndrome, emesis, depression, anxiety, manic depressive psychosis or schizophrenia.

**11.** A pharmaceutical agent comprising (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine and an anti-cholinergic drug in combination.

**12.** The pharmaceutical agent of claim 7 or 11, wherein the anti-cholinergic drug is oxybutynin, propiverine, darifenacin, tolterodine, temiverine, trospium chloride or a salt thereof.

**13.** The pharmaceutical agent of claim 11, which is a prophylactic or therapeutic agent of urinary frequency, urinary incontinence, asthma, chronic obstructive pulmonary disease, rheumatoid arthritis, osteoarthritis, pain, cough, irritable bowel syndrome, emesis, depression, anxiety, manic depressive psychosis or schizophrenia.

**14.** A method for the prophylaxis or treatment of urinary frequency, urinary incontinence, asthma, chronic obstructive pulmonary disease, rheumatoid arthritis, osteoarthritis, pain, cough, irritable bowel syndrome, emesis, depression, anxiety, manic depressive psychosis or schizophrenia, which comprises administering an effective amount of (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g] [1,7]naphthyridine and an effective amount of an anti-cholinergic drug in combination to a mammal.

**15.** Use of (9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g] [1,7]naphthyridine and an anti-cholinergic drug for the production of a prophylactic or therapeutic agent of urinary frequency, urinary incontinence, asthma, chronic obstructive pulmonary disease, rheumatoid arthritis, osteoarthritis, pain, cough, irritable bowel syndrome,emesis, depression, anxiety, manic depressive psychosis or schizophrenia.

**16.** A commercial package comprising a pharmaceutical agent of claim 1 and written matter associated therewith, the written matter stating that the pharmaceutical agent can or should be used for the prophylaxis or treatment of urinary frequency, urinary incontinence, asthma, chronic obstructive pulmonary disease, rheumatoid arthritis, osteoarthritis, pain, cough, irritable bowel syndrome, emesis, depression, anxiety, manic depressive psychosis or schizophrenia.

## INTERNATIONAL SEARCH REPORT

International application No.
PCT/JP01/11231

A. CLASSIFICATION OF SUBJECT MATTER
Int.Cl[7] A61K45/06, 31/551, 31/221, 31/4468, A61P11/00, 29/00, 19/02, 11/14, 1/00, 1/08, 25/24, 25/22, 25/18, 43/00

B. FIELDS SEARCHED
Minimum documentation searched (classification system followed by classification symbols)
Int.Cl[7] A61K45/06, 31/551, 31/221, 31/4468, A61P11/00, 29/00, 19/02, 11/14, 1/00, 1/08, 25/24, 25/22, 25/18, 43/00

Documentation searched other than minimum documentation to the extent that such documents are included in the fields searched

Electronic data base consulted during the international search (name of data base and, where practicable, search terms used)
CAPLUS(STN), MEDLINE(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| Y | FOULON D. M. et al., NK1 and NK2 receptors mediate tachykinin and resiniferatoxin-induced bronchospasm in guinea pigs, Am. Rev. Respir. Dis., 1993, Vol.148, No.4 Pt.1, pages 915 to 921 | 1-8,10-13, 15,16 |
| Y | MAGGI C. A. et al., Tachykinin receptors and airway pathophysiology, Eur. Respir. J., 1993, Vol.6, No.5, pages 735 to 742 | 1-8,10-13, 15,16 |
| Y | EP, 733632, A1 (Takeda Chemical Industries Ltd.), 25 September, 1996 (25.09.96), & US 5786352 A & JP 9-263585 A & JP 9-263587 A & JP 10-109989 A | 1-8,10-13, 15,16 |
| Y | JP, 11-322748, A (Takeda Chemical Industries, Ltd.) 24 November, 1999 (24.11.99), (Family: none) | 1-8,10-13 15,16 |
| Y | JP, 11-43489, A (Takeda Chemical Industries, Ltd.), 16 February, 1999 (16.02.99), (Family: none) | 1-8,10-13, 15,16 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 March, 2002 (19.03.02) | 09 April, 2002 (09.04.02) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP01/11231

C (Continuation). DOCUMENTS CONSIDERED TO BE RELEVANT

| Category* | Citation of document, with indication, where appropriate, of the relevant passages | Relevant to claim No. |
|---|---|---|
| Y | DOI T. et al., Effects of TAK-637, a tachykinin receptor antagonist, on lower urinary tract function in the guinea pig, Eur. J. Pharmacol., 1999, Vol.383, No.3, pages 297 to 303 | 1-8,10-13, 15,16 |
| Y | DOI T. et al., Effects of TAK-637, a tacchykinin receptor antagonist, on the micturition reflex in guinea pigs, Eur. J. Pharmacol., 2000, Vol.385, No.3, pages 241 to 246 | 1-8,10-13, 15,16 |

Form PCT/ISA/210 (continuation of second sheet) (July 1998)

**INTERNATIONAL SEARCH REPORT**

International application No.
PCT/JP01/11231

**Box I Observations where certain claims were found unsearchable (Continuation of item 1 of first sheet)**

This international search report has not been established in respect of certain claims under Article 17(2)(a) for the following reasons:

1. [X] Claims Nos.: 9, 14
because they relate to subject matter not required to be searched by this Authority, namely:
(See extra sheet)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:
(See extra sheet)

1. [X] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[X] No protest accompanied the payment of additional search fees.

Form PCT/ISA/210 (continuation of first sheet (1)) (July 1998)

**INTERNATIONAL SEARCH REPORT**

International application No.
PCT/JP01/11231

Continuation of Box No. I of Continuation of first sheet (1)

The inventions as set forth in claims 9 and 14 pertain to methods for treatment of the human body by therapy and thus relate to a subject matter which this International Searching Authority is not required, under the provisions of Article 17(2)(a)(i) of the PCT and Rule 39(iv) of the Regulations under the PCT, to search.

Continuation of Box No. II of Continuation of first sheet (1)

Observations where unity of invention is lacking (Continuation)

Independent claims in the present application are summarized as follows.

The invention as set forth in claim 1 in the present application relates to a "drug for unspecified use" which comprises a combination of "a compound, its salt or a prodrug thereof", i.e., a specific compound represented by the formula given therein with "an NK-2 receptor antagonist".

The invention as set forth in claim 7 in the present application relates to a "drug for unspecified use" which comprises a combination of "an NK-1 receptor antagonist", the structure of which is not specified, with "an NK-2 receptor antagonist" further combined with a cholinolytic.

The invention as set forth in claim 8 in the present application relates to a preparation for restricted use (i.e., preventives for urinary frequency, urinary incontinence, etc.) which comprises a combination of "an NK-1 receptor antagonist", the structure of which is not specified, with "an NK-2 receptor antagonist".

Although the subject of the invention as set forth in claim 10 in the present application resides in urinary frequency and urinary incontinence, it is not restricted thereto but relates to the use of "a compound (I), its salt or a prodrug thereof", which is a specific compound represented by the formula given therein, with "an NK-2 receptor antagonist" for producing preventives and remedies for asthma, chronic obstructive pulmonary disease, rheumatoid arthritis, arthritis deformans, pain, cough, irritable bowel syndrome, vomiting, depression, anxiety, manic-depression or schizophrenia.

The invention as set forth in claim 11 in the present application relates to a "drug for unspecified use" which comprises a combination of a single specific compound "(9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine] described therein with a cholinolytic.

Although the subject of the invention as set forth in claim 15 in the present application resides in urinary frequency and urinary incontinence, it is not restricted thereto but relates to the use of a single specific compound "(9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine] described therein with a cholinolytic for producing preventives and remedies for asthma, chronic obstructive pulmonary disease, rheumatoid arthritis, arthritis deformans, pain, cough, irritable bowel syndrome, vomiting, depression, anxiety, manic-depression or schizophrenia.

The invention as set forth in claim 16 in the present application relates to a commercial package containing the above-described drug and a "document" indicating that it is usable or to be used in preventing and treating asthma, chronic obstructive pulmonary disease, rheumatoid arthritis, arthritis deformans, pain, cough, irritable bowel syndrome, vomiting, depression, anxiety, manic-depression or schizophrenia, in addition to urinary frequency and urinary incontinence.

Form PCT/ISA/210 (extra sheet) (July 1998)

## INTERNATIONAL SEARCH REPORT

International application No.
PCT/JP01/11231

Based on the statement in the description of the present application, it is understood that the specific compound "(9R)-7-[3,5-bis(trifluoromethyl)benzyl]-6,7,8,9,10,11-hexahydro-9-methyl-5-(4-methylphenyl)-6,13-dioxo-13H-[1,4]diazocino[2,1-g][1,7]naphthyridine] as described above is involved in the scope of the above-described compound (I). It is also understood that the compound (I) belongs to the category of "NK-1 receptor antagonist".

As stated in the document presented in "C. DOCUMENTS CONSIDERED TO BE RELEVANT" in this international search report, it had been publicly known prior to the priority date of the present application that compounds serving both as an NK-1 receptor antagonist and an NK-2 receptor antagonist are usable in preventing and treating asthma, chronic obstructive pulmonary disease, rheumatoid arthritis, arthritis deformans, pain, cough, digestive diseases, vomiting, neurodegenerative diseases, depression, in addition to vesical function disorders including urinary frequency and urinary incontinence.

Also, it had been publicly known prior to the priority date of the present application that the simultaneous administration of an NK-1 receptor antagonist and an NK-2 receptor antagonist exerts a synergistic effect on bronchoconstriction.

*Considering these prior arts, the inventions of the present application may* be roughly classified as follows.

(1) Provision of drugs by combining an NK-1 receptor antagonist having a specific structure with an NK-2 receptor antagonist.

(2) Provision of drugs by combining an NK-1 receptor antagonist having a specific structure with a cholinolytic.

(3) Provision of drugs by combining an unspecified NK-1 receptor with an NK-2 receptor antagonist combined with a cholinolytic.

(4) Provision of specific medicinal use ( i.e., preventives for urinary frequency and urinary incontinence) of a combination of an unspecified NK-1 receptor with an NK-2 receptor antagonist optionally combined with a cholinolytic.

Although these groups of inventions each aims at establishing a synergistic effect, means for establishing the purpose or the subjects of the purpose are not identical with each other.

Such being the case, these groups of inventions are not considered as relating to a group of inventions so linked as to form a single general inventive concept. It is publicly known to use in preventing and treating vomiting, neurodegenerative diseases, etc.

Form PCT/ISA/210 (extra sheet) (July 1998)